(19) Europäisches Patentamt
European Patent Office
Office européen des brevets

(11) **EP 2 339 329 A2**

(12) **EUROPÄISCHE PATENTANMELDUNG**

(43) Veröffentlichungstag:
**29.06.2011 Patentblatt 2011/26**

(51) Int Cl.:
**G01N 21/47** (2006.01)   **A61B 5/00** (2006.01)
**A61B 3/13** (2006.01)   **A61B 3/12** (2006.01)
**G01B 9/02** (2006.01)

(21) Anmeldenummer: **11002235.7**

(22) Anmeldetag: **21.02.2007**

(84) Benannte Vertragsstaaten:
**AT BE BG CH CY CZ DE DK EE ES FI FR GB GR HU IE IS IT LI LT LU LV MC NL PL PT RO SE SI SK TR**

(62) Dokumentnummer(n) der früheren Anmeldung(en) nach Art. 76 EPÜ:
**07102825.2 / 1 962 082**

(71) Anmelder: **Agfa HealthCare N.V.**
**2640 Mortsel (BE)**

(72) Erfinder: **Nebosis, Rainer, Dr.**
**81541 München (DE)**

(74) Vertreter: **Linsmeier, Josef**
**Agfa-Gevaert HealthCare GmbH**
**Intellectual Property**
**Tegernseer Landstraße 161**
**81539 München (DE)**

Bemerkungen:
Diese Anmeldung ist am 18-03-2011 als Teilanmeldung zu der unter INID-Code 62 erwähnten Anmeldung eingereicht worden.

(54) **System und Verfahren zur optischen Kohärenztomographie**

(57) Die Erfindung betrifft ein System sowie ein entsprechendes Verfahren zur optischen Kohärenztomographie mit einem Interferometer (10, 20) zur Ausgabe von Licht, mit dem eine Probe (1) bestrahlt wird, wobei das Interferometer (10, 20) einen Strahlteiler (13, 24) und mindestens einen Reflektor (11, 12, 25) umfasst, und einem Detektor (30) mit einer ersten Anzahl von in einer ersten Fläche angeordneten Detektorelementen zur Erfassung von Licht, welches von der Probe (1) reflektiert wird.

Um auf einfachere und schnellere Weise, insbesondere in Echtzeit, Bilder einer Probe (1) aufnehmen zu können, ist das System in einem ersten Modus, insbesondere einem ersten Echtzeitmodus, betreibbar, in welchem von der Probe (1) reflektiertes Licht nur von einer zweiten Anzahl von Detektorelementen des Detektors (30) erfasst und in entsprechende Detektorsignale umgewandelt wird, wobei die zweite Anzahl der Detektorelemente kleiner ist als die erste Anzahl der Detektorelemente. Darüber hinaus ist das System in einem zweiten Modus, insbesondere einem zweiten Echtzeitmodus, betreibbar, in welchem während einer Veränderung des optischen Abstandes des Reflektors (11, 12, 25) zum Strahlteiler (13, 24) das von der Probe (1) reflektierte Licht von den Detektorelementen des Detektors (30) mehrmals, insbesondere höchstens fünfmal, erfasst wird, wodurch ein zweidimensionaler Schnitt durch ein Raumelement der Probe (1) in einer vorgegebenen Tiefe der Probe (1) erhalten wird.

Fig. 1

**Beschreibung**

Die Anmeldung betrifft ein System sowie ein entsprechendes Verfahren zur optischen Kohärenztomographie gemäß dem Oberbegriff des Anspruchs 1 bzw. 15.

Die optische Kohärenztomographie (OCT) ist eine Methode, um lichtstreuende Proben in ihrem Inneren zu vermessen. Biologisches Gewebe ist aufgrund seiner lichtstreuenden Eigenschaften für die diagnostische Untersuchung mittels OCT besonders geeignet. Da die OCT mit relativ geringen Lichtintensitäten auskommt und die Wellenlängen des verwendeten Lichts zumeist in nahen Infrarotbereich (750 nm bis 1350 nm) liegen, stellt sie im Gegensatz zur ionisierenden Röntgendiagnostik für biologisches Gewebe keine Strahlenbelastung dar. Sie ist somit besonders für die Medizin von Bedeutung und grob vergleichbar mit der Ultraschalldiagnostik. Anstelle von Schall wird bei der OCT breitbandiges Licht, welches eine sehr kurze Kohärenzlänge aufweist, verwendet. Die Laufzeiten des an unterschiedlichen Grenzschichten in der Probe reflektierten Lichts werden mit Hilfe eines Interferometers erfasst. Mit der OCT sind typischerweise um ein bis zwei Größenordnungen höhere Auflösungen als mit Ultraschall zu erreichen, jedoch ist die erzielbare Vermessungstiefe deutlich kleiner. Die gewonnenen Querschnittsbilder reichen aufgrund von optischer Streuung nur bis zu einer Tiefe von wenigen Millimetern in das Gewebe hinein. Die derzeit wichtigsten Anwendungsbereiche der OCT liegen in der Ophthalmologie, der Dermatologie sowie der Krebsdiagnose. Es existieren allerdings auch einige nichtmedizinische Anwendungen, wie z.B. in der Werkstoffprüfung.

Aus W. Y. Oh et al., OPTICS EXPRESS Vol. 14, No. 19 (2006) 8675 - 8684 ist ein gattungsgemäßes System bekannt, bei welchem die zu untersuchende Probe auf einer von einem Computer gesteuerten beweglichen Bühne angeordnet ist. Um ein dreidimensionales Bild der Probe zu erhalten, werden bei unterschiedlichen Positionen der Bühne zweidimensionale Bilder der Probe aufgenommen. Wegen des mit der Realisierung der beweglichen Bühne verbundenen mechanischen Aufwands und hohen Platzbedarfs ist dieses System für endoskopische Anwendungen nur bedingt geeignet. Darüber hinaus ist das Erfassen von zweidimensionalen Bildern der Probe bei unterschiedlichen Probenpositionen relativ zeitaufwändig, was bei Untersuchungen an lebenden Objekten, die eine bestimmte Ruheposition im Allgemeinen nur kurzzeitig halten können, zu einem Verwackeln der Bildinformation beitragen kann.

Es ist Aufgabe der Erfindung, ein System sowie ein entsprechendes Verfahren zur optischen Kohärenztomographie anzugeben, bei welchem auf einfachere und schnellere Weise, insbesondere in Echtzeit, Bilder einer Probe aufgenommen werden können.

Diese Aufgabe wird durch das System gemäß Anspruch 1 bzw. das entsprechende Verfahren gemäß Anspruch 15 dadurch gelöst, dass der Detektor eine erste Anzahl von in einer ersten Fläche angeordneten Detektorelementen zur Erfassung von Licht aufweist und das System in einem ersten Modus, insbesondere einem ersten Echtzeitmodus, betrieben werden kann bzw. betrieben wird, in welchem von der Probe reflektiertes Licht nur von einer zweiten Anzahl von Detektorelementen des Detektors erfasst und in entsprechende Detektorsignale umgewandelt wird, wobei die zweite Anzahl der Detektorelemente kleiner ist als die erste Anzahl der Detektorelemente des Detektors. Ferner ist bzw. wird das System in einem zweiten Modus, insbesondere einem zweiten Echtzeitmodus, betreibbar bzw. betrieben, in welchem während einer Veränderung des optischen Abstandes des Reflektors zum Strahlteiler das von der Probe reflektierte Licht von den Detektorelementen des Detektors mehrmals, insbesondere höchstens fünfmal, erfasst wird, wodurch ein zweidimensionaler Schnitt durch ein Raumelement der Probe in einer vorgegebenen Tiefe der Probe erhalten wird.

Die Erfindung basiert auf dem Gedanken, einen Detektor zu verwenden, der im ersten Betriebsmodus so betrieben werden kann, dass lediglich eine Teilfläche (ein sog. Window of Interest, WOI) des Detektors für Licht sensitiv ist, wobei lediglich das von Detektorelementen dieser Teilfläche erfasste Licht in entsprechende Detektorsignale umgewandelt wird. Durch die Reduktion der sensitiven Detektorfläche und die damit einhergehende Beschränkung der Bilderfassung auf einen ausgewählten Ausschnitt der Probe werden die für die Erfassung von Licht sowie dessen Umwandlung in entsprechende Detektorsignale erforderlichen Zeiten erheblich verkürzt, so dass wesentlich mehr Bilder pro Zeiteinheit erzeugt werden können als im Vollbild-Modus. Die Reduktion der sensitiven Fläche des Detektors führt somit zu einer Erhöhung der Bildrate.

Darüber hinaus können im zweiten Betriebsmodus zweidimensionale Tomogramme aus einer bestimmten Tiefe des betrachteten Raumelements der Probe erzeugt werden, wobei die Tiefe frei wählbar ist. Hierbei wird die gesamte Detektorfläche des Detektors für die Erfassung des von der Probe reflektierten Lichts und dessen Umwandlung in entsprechende Detektorsignale genutzt, wobei jedoch nur jeweils maximal fünf Kamerabilder zur Berechnung eines Tomogramms herangezogen werden. Dazu wird der Referenzspiegel im Interferometer mit einer Amplitude von etwa 1 μm periodisch bewegt, während bis zu fünf Kamerabilder aufgenommen werden, die dann zu einem OCT-Bild verrechnet werden. Auf diese Weise können Tomogramme mit hoher Wiederholungsrate erzeugt werden.

Die Erfindung ermöglicht auf einfache Weise die Aufnahme von Bildern mit hohen Raten von etwa 5 bis 10 Bildern pro Sekunde und erlaubt somit die Untersuchung einer Probe in Echtzeit.

Durch die verschiedenen Betriebsmodi kann das OCT-System darüber hinaus eine Reihe unterschiedlicher Anforderungen erfüllen. Die Funktionalitäten bei der Untersuchung von Proben, beispielsweise beim Auffinden relevanter Stellen in der Probe, werden dadurch erheblich erweitert.

Vorzugsweise beträgt die zweite Anzahl der Detektorelemente höchstens ein Viertel der ersten Anzahl der Detektorelemente. Dadurch wird die Bildrate gegenüber dem Vollbild-Modus um mindestens den Faktor 4 erhöht.

Es ist außerdem bevorzugt, dass die zweite Anzahl der Detektorelemente in einer zweiten Fläche angeordnet ist, welche eine zusammenhängende Teilfläche der ersten Fläche, d.h. der Gesamtfläche, des Detektors bildet. Insbesondere weist die erste Fläche des Detektors eine erste Breite und eine erste Länge und die zweite Fläche eine zweite Breite und eine zweite Länge auf, wobei die erste und die zweite Länge im Wesentlichen identisch sind und die zweite Breite kleiner ist als die erste Breite. Hierdurch wird die Auswahl einer schmalen, länglichen Teilfläche mit kleiner Breite und maximaler Länge ermöglicht.

Das Interferometer umfasst einen Strahlteiler und mindestens einen Reflektor, dessen optischer Abstand zum Strahlteiler um einen optischen Weg veränderbar ist. Der optische Abstand des Reflektors zum Strahlteiler ist gegeben durch den räumlichen Abstand des Reflektors zum Strahlteiler, welcher mit dem Brechungsindex des zwischen dem Reflektor und dem Strahlteiler befindlichen Mediums multipliziert wird. Bei einer Ausgestaltung des Interferometers als sog. Freistrahl-Interferometer, bei welchem sich zwischen dem Reflektor und dem Strahlteiler Luft oder Vakuum befindet und der Brechungsindex etwa gleich 1 ist, ist der optische Abstand des Reflektors sowie der optische Weg, um welchen der optische Abstand verändert wird, identisch mit dessen räumlichem Abstand bzw. räumlichem Weg. Die makroskopische Veränderung des optischen Abstands des Reflektors wird in diesem Fall durch eine makroskopische Bewegung des Reflektors um einen räumlichen Weg realisiert, welcher wesentlich größer ist als die mittlere Weglänge des in das Interferometer eingekoppelten Lichts. Alternativ kann bei einer Ausgestaltung des Interferometers als sog. Faser-Interferometer zwischen dem Reflektor und dem Strahlteiler ein lichtleitendes Element, insbesondere eine Lichtleitfaser, vorgesehen sein, dessen optische Länge gezielt um einen optischen Weg veränderbar ist. Solche Lichtleitfasern werden auch als Fiber Stretcher bezeichnet. In diesem Fall ist der optische Abstand sowie der optische Weg, um welchen der optische Abstand verändert wird, durch das Produkt aus dem räumlichen Abstand bzw. dem räumlichen Weg, um welchen der Abstand verändert wird, und dem Brechungsindex des Lichtleitelements, welcher typischerweise im Bereich um 1,5 liegt, gegeben.

In einer weiteren Ausführung der Erfindung ist der optische Abstand des Reflektors zum Strahlteiler um einen optischen Weg veränderbar, der wesentlich größer ist als die mittlere Wellenlänge von in das Interferometer eingekoppeltem Licht. Auf diese Weist ist ein sog. Tiefenscan oder z-Scan realisierbar, indem der optische Abstand des Reflektors zum Strahlteiler um makroskopische optische Wege von typischerweise 0,1 mm bis zu mehreren Millimetern verändert wird. Mit zunehmendem bzw. abnehmendem optischen Abstand des Reflektors zum Strahlteiler "wandern" die Tiefenbereiche, in welchen die Kohärenzbedingung für Interferenz erfüllt ist, durch die Probe. Das von einzelnen Ebenen in unterschiedlichen Tiefen der Probe reflektierte Licht kann hierbei sukzessive erfasst, ausgewertet und schließlich zu einem Bild der Probe zusammengesetzt werden. Auf eine bewegliche Bühne zur Wahl der jeweiligen Tiefe der Probe, in welcher ein Bild aufgenommen werden soll, kann hierdurch verzichtet werden. Gleichzeitig wird dadurch die Erfassung mehrerer Bilder in unterschiedlichen Tiefen der Probe wesentlich beschleunigt.

Die mittlere Wellenlänge des in das Interferometer eingekoppelten Lichts liegt typischerweise im infraroten Spektralbereich, vorzugsweise zwischen 750 und 1350 nm. Im Falle einer breitbandigen Lichtquelle liegt die mittlere Wellenlänge des Lichts vorzugsweise in einem spektralen Bereich, in welchem die Lichtquelle ein Intensitätsmaximum aufweist. Alternativ dazu ist die mittlere Wellenlänge durch einen Mittelwert aus allen von der Lichtquelle emittierten Wellenlängen gegeben. Vorzugsweise liegt die mittlere Wellenlänge des in das Interferometer eingekoppelten in einem Wellenlängenbereich, in welchem der Detektor eine sehr hohe, insbesondere die höchste, Empfindlichkeit aufweist.

Im ersten Modus des Systems wird während der Veränderung des optischen Abstands des makroskopisch beweglichen Reflektors zum Strahlteiler um den optischen Weg das von der Probe reflektierte Licht nur von der zweiten Anzahl von Detektorelementen des Detektors mehrmals erfasst, wodurch mehrere zweidimensionale Tiefenschnitte durch ein Raumelement der Probe erhalten werden. Die Anzahl der Tiefenschnitte entspricht hierbei der Anzahl der Detektorelemente entlang der zweiten Breite der zweiten Fläche der Detektorelemente. Umfasst die zweite Fläche des Detektors z.B. 640 x 4 sensitive Detektorelemente, so werden insgesamt vier Tiefenschnitte durch die Probe erhalten.

Im zweiten Modus des Systems wird der optische Abstand eines weiteren Reflektors zum Strahlteiler um einen weiteren optischen Weg verändert, welcher höchstens das Zehnfache der mittleren Wellenlänge des in das Interferometer eingekoppelten Lichts beträgt. Während der Veränderung des optischen Abstands dieses mikroskopisch beweglichen Reflektors zum Strahlteiler wird das von der Probe reflektierte Licht von den Detektorelementen des Detektors mehrmals, insbesondere bis zu fünfmal, erfasst, wodurch ein Bild eines zweidimensionalen Schnitts in einer bestimmten Tiefe der Probe erhalten wird. Die jeweilige Tiefe der Probe, aus welcher das Bild eines zweidimensionalen Schnittes erhalten wird, wird hierbei durch den optischen Abstand des makroskopisch beweglichen Reflektors zum Strahlteiler vorgegeben.

Bei dieser Ausführung werden also sowohl der makroskopisch als auch der mikroskopisch bewegliche Reflektor bewegt. Durch die Wahl des Abstands des makroskopisch beweglichen Reflektors vom Strahlteiler wird die Tiefe vorgegeben, in welcher ein zweidimensionaler Schnitt aufgenommen werden soll. Die eigentliche Aufnahme des für die Bilderzeugung relevanten Lichts an bis zu fünf Zeitpunkten erfolgt dann während der Bewegung des mikroskopisch beweglichen Reflektors. Der makroskopisch bewegliche Reflektor befindet sich währenddessen in einem festen optischen Abstand zum Strahlteiler. Hierdurch wird auf einfache und schnelle Weise die Erfassung von zweidimensionalen Schnitten durch die Probe in Echtzeit ermöglicht.

In einem dritten Modus des Systems wird während der Veränderung des optischen Abstands des makroskopisch beweglichen Reflektors zum Strahlteiler um den optischen Weg das von der Probe reflektierte Licht von den Detektorelementen des Detektors mehrmals erfasst, wodurch das von mehreren zweidimensionalen Schnitten in unterschiedlichen Tiefen der Probe reflektierte Licht sukzessive erfasst wird. Hierbei wandern die Tiefenbereiche, in welchen die Kohärenzbedingung für Interferenz erfüllt ist, synchron mit der makroskopischen Änderung des optischen Abstands des makroskopisch beweglichen Reflektors vom Strahlteiler durch die Probe, so dass das von einzelnen Ebenen in unterschiedlichen Tiefen der Probe reflektierte Licht sukzessive erfasst, ausgewertet und schließlich zu einem dreidimensionalen Bild der Probe zusammengesetzt werden kann.

Vorzugsweise ist ein Probenobjektiv vorgesehen, durch welches vom Interferometer ausgegebenes Licht in einem auf oder in der Probe liegenden Fokus fokussiert wird, wobei während einer Veränderung des optischen Abstands des makroskopisch beweglichen Reflektors zum Strahlteiler das in mehreren unterschiedlichen Tiefen der Probe jeweils reflektierte Licht vom Detektor erfasst wird und gleichzeitig die Abbildungseigenschaften des Probenobjektivs in der Weise gesteuert werden, dass der Fokus im Bereich der jeweiligen Tiefe der Probe liegt. Durch die Veränderung des optischen Abstands des Reflektors zum Strahlteiler werden - wie bereits oben ausgeführt - aus unterschiedlichen Tiefen der Probe herrührende Interferenzmuster erfasst. Synchron dazu wird der Fokus des Probenobjektivs in der Weise verändert, dass er in den Bereich der jeweiligen Tiefe der Probe fällt, von welchem gerade ein Interferenzmuster erfasst wird. Auf diese Weise wird der Fokus des in die Probe eingestrahlten Lichts während des Tiefenscans nachgeführt, was daher auch als "Fokus Tracking" bezeichnet werden kann. Durch die Anpassung des Fokus des Probenobjektivs an die jeweilige auszulesende Tiefe während eines Tiefenscans wird erreicht, dass das aus einer bestimmten Tiefe der Probe erhaltene Interferenzmuster stets mit der größtmöglichen Schärfe auf den Detektor abgebildet und erfasst werden kann.

Es ist außerdem bevorzugt, die Intensität von Licht, welches in das Interferometer eingekoppelt oder vom Interferometer ausgegeben wird, mit einer Modulationsfrequenz zu modulieren. Durch die Modulation der Intensität des eingekoppelten bzw. ausgegebenen Lichts wird am Detektor anstelle eines hochfrequenten Interferenzmusters mit einer Vielzahl von Perioden eine niederfrequente Schwebung zwischen der Modulation und dem zu erfassenden Interferenzmuster erhalten, wobei die niederfrequente Schwebung deutlich weniger Perioden aufweist als das hochfrequente Interferenzmuster. Bei der Erfassung dieser Schwebung durch den Detektor sind daher deutlich weniger Abtastzeitpunkte pro Zeiteinheit erforderlich als bei einer Erfassung des Interferenzmusters ohne die Modulation der Lichtintensität. Dies trägt zu einer weiteren Beschleunigung der Bilderfassung und damit zu einem besonders zuverlässigen Echtzeitbetrieb bei.

Alternativ ist ein Detektorsystem vorgesehen, welches den Detektor umfasst, wobei die Empfindlichkeit des Detektorsystems für das von der Probe reflektierte und auf den Detektor treffende Licht mit einer Modulationsfrequenz $f_M$ moduliert wird. Das von der Probe reflektierte und auf den Detektor treffende Licht überlagert sich mit der modulierten Empfindlichkeit des Detektorsystems, so dass der Detektor bei der Erfassung des auf den Detektor treffenden Interferenzmusters anstelle eines hochfrequenten Interferenzsignals mit einer Vielzahl von Perioden ein niederfrequentes Schwebungssignal erzeugt, welches deutlich weniger Perioden aufweist als das hochfrequente Interferenzsignal. Bei der Erfassung dieser Schwebung sind daher deutlich weniger Abtastzeitpunkte pro Zeiteinheit erforderlich als bei einer Erfassung des hochfrequenten Interferenzsignals ohne die Modulation der Empfindlichkeit des Detektorsystems. Auf diese Weise wird erreicht, dass bei einer gegebenen maximalen Abtastrate deutlich kürzere Zeiten für die Erfassung eines aus einer bestimmten Tiefe der Probe erhaltenen Interferenzmusters benötigt werden. Die pro Zeiteinheit erfassbare Anzahl von Interferenzsignalen wird dadurch deutlich erhöht. Hierdurch wird die Bilderfassung weiter beschleunigt und damit ein besonders zuverlässiger Echtzeitbetrieb ermöglicht.

Vorzugsweise ist die Modulationsfrequenz $f_M$ ungleich der Dopplerfrequenz $f_D$, wobei die Dopplerfrequenz $f_D$ durch das Zweifache des Verhältnisses der Geschwindigkeit v der Veränderung des optischen Abstands des makroskopisch beweglichen Reflektors bzw. mikroskopisch beweglichen Reflektors zum Strahlteiler zur mittleren Wellenlänge $\lambda_0$ des in das Interferometer eingekoppelten Lichts gegeben ist: $f_M \neq f_D = 2 \cdot v/\lambda_0$. Hierdurch wird gewährleistet, dass eine niederfrequente Schwebung unabhängig von der jeweiligen Phasenlage der Modulation und des Interferenzmusters erhalten wird. Die erfindungsgemäße Beschleunigung der Erfassung von Interferenzmustern wird hierdurch mit hoher Zuverlässigkeit erreicht.

Im Sinne der Erfindung ist unter einer Bestrahlung der Probe mit dem vom Interferometer ausgegebenen Licht zu verstehen, dass das vom Interferometer, das den beweglichen Reflektor umfasst, ausgegebene Licht direkt auf die Probe trifft oder erst nach Durchlaufen eines weiteren Interferometers, welches zwischen dem Interferometer und der Probe angeordnet ist, auf die Probe trifft.

Im Sinne der Erfindung ist unter einer Erfassung des von der Probe, insbesondere in unterschiedlichen Tiefen der Probe, reflektierten Lichts durch den Detektor bzw. die Detektorelemente zu verstehen, dass der Detektor bzw. die Detektorelemente das Licht von Interferenzerscheinungen erfassen, die bei einer Überlagerung des von der Probe, insbesondere in unterschiedlichen Tiefen der Probe, reflektierten Lichts mit dem an einem Referenzspiegel reflektierten Licht entstehen. Die Überlagerung des Lichts kann hierbei entweder in dem Interferometer, das den beweglichen Reflektor umfasst, oder in einem weiteren Interferometer erfolgen.

Die Erfindung sowie weitere vorteilhafte Ausgestaltungen der Erfindung werden nachfolgend anhand von Figuren näher erläutert. Es zeigen:

Fig. 1        ein Ausführungsbeispiel des erfindungsgemäßen OCT-Systems;

Fig. 2 a-b)   zwei Raumelemente einer Probe mit einzelnen Schnitten;

Fig. 3 a-b)   zwei Querschnitte durch die Probe und den Probenarm des zweiten I nterferometers;

Fig. 4        einen Querschnitt durch die optischen Komponenten des zweiten Interferometers;

Fig. 5        Interferenzsignale und deren Auswertung bei der automatischen Kalibrierung der Fokusnachführung;

Fig. 6 a-c)   Interferenzsignale und deren Einhüllende bei unmodulierter und modulierter Intensität des in das erste Interferometer eingekoppelten Lichts;

Fig. 7        ein Beispiel für eine elektrische Schaltung zur Modulation der Empfindlichkeit des Detektors;

Fig. 8        einen beispielhaften Aufbau eines sog. Linnik-Interferometers;

Fig. 9 a-c)   drei unterschiedliche Positionen des Probenobjektivs und die jeweils erhaltenen Interferenzmuster;

Fig. 10 a-b)  jeweils einen Ausschnitt aus einem Längsschnitt durch die Multimode-Faser des ersten Lichtleiters im Bereich der Eingangsebene;

Fig. 11       einen Ausschnitt aus einem Querschnitt durch das Faserbündel des zweiten Lichtleiters sowie einen vergrößert dargestellten Teilbereich dieses Ausschnitts;

Fig. 12       einen Ausschnitt der Detektorfläche;

Fig. 13       die Detektorfläche und die Eintritts- und Austrittsfläche des zweiten Lichtleiters;

Fig. 14 a-b)  zwei Beispiele für die Ausgestaltung des zweiten Lichtleiters im Querschnitt;

Fig. 15       ein Interferenzmuster sowie einen Ausschnitt aus dem Interferenzmuster im Vergleich mit den Einzelfasern des zweiten Lichtleiters;

Fig. 16       einen Ausschnitt aus einem Längsschnitt durch das Faserbündel des zweiten Lichtleiters im Bereich der Eintrittsfläche;

Fig. 17       eine Detektorfläche im ersten Betriebsmodus;

Fig. 18       ein Raumelement der Probe mit Tiefenschnitten; und

Fig. 19       ein Raumelement der Probe mit einem zweidimensionalen Tomogramm in einer bestimmten Tiefe.

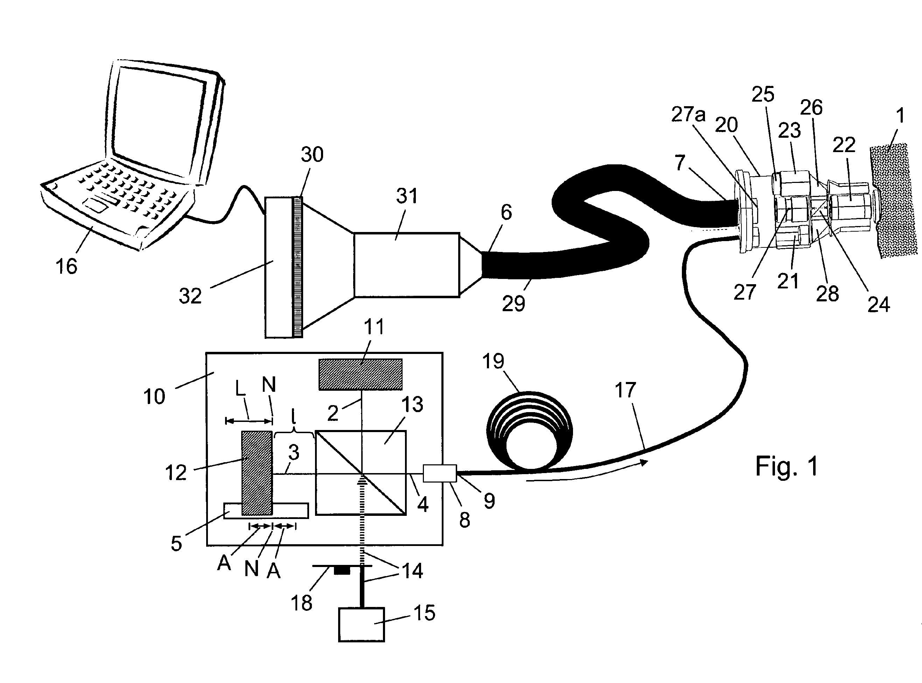

Fig. 1 zeigt ein Ausführungsbeispiel des erfindungsgemäßen Systems zur OCT. Die hier gewählte Darstellung der einzelnen Komponenten des Systems ist stark schematisiert und nicht maßstabsgetreu.

Ein erstes Interferometer 10 weist einen ortsfest angeordneten ersten Referenzspiegel 11, einen beweglichen zweiten Referenzspiegel 12 und einen ersten Strahlteiler 13 auf. Licht 14 einer Lichtquelle 15 wird in das erste Interferometer 10 eingekoppelt, vom ersten Strahlteiler 13 in einen ersten Teilstrahl 2 in Richtung des ortsfest angeordneten ersten Referenzspiegels 11 und einen zweiten Teilstrahl 3 in Richtung des beweglichen zweiten Referenzspiegels 12 geteilt. Die beiden Teilstrahlen 2 bzw. 3 werden vom ortsfesten ersten bzw. beweglichen zweiten Referenzspiegel 11 bzw. 12 reflektiert und überlagern sich im ersten Strahlteiler 13 zu einem dritten Teilstrahl 4, der im Bereich des Ausgangs 8 des ersten Interferometers 10 in einen ersten Lichtleiter 17 eingekoppelt, von diesem zu einem zweiten Interferometer 20 geleitet und dort in einen Beleuchtungsarm 21 des zweiten Interferometers 20 eingekoppelt wird.

Das in das erste Interferometer 10 eingekoppelte Licht 14 wird durch den beschriebenen Strahlengang in Verbindung mit der Bewegung des zweiten Referenzspiegels 12 spektral moduliert und verlässt das erste Interferometer 10 in Form des dritten Teilstrahls 4, der in das zweite Interferometer 20 eingekoppelt wird. Daher kann das erste Interferometer 10 auch als Prä-Modulator bezeichnet werden.

Das zweite Interferometer 20 dient als Sensor- oder Messkopf, welcher von einem Bediener, beispielsweise einem Arzt, manuell mit der zu untersuchenden Probe 1, insbesondere einem biologischen Gewebe, in Verbindung gebracht und ggf. auf dieser geführt wird. Der Messkopf ist hierbei so kompakt aufgebaut, dass seine Länge vorzugsweise der eines üblichen Schreibgerätes, wie z.B. eines Füllfederhalters, entspricht.

Um das zweite Interferometer 20 derart kompakt auszugestalten, sind die optischen Achsen des Beleuchtungsarms 21 sowie eines Referenzarms 23, in welchem ein dritter Referenzspiegel 25 ortsfest angeordnet ist, jeweils um 90° gegenüber der herkömmlichen senkrechten Anordnung der beiden optischen Achsen (siehe erstes Interferometer 10) gekippt und verlaufen parallel zueinander. Zur Umlenkung der Lichtstrahlen aus dem Beleuchtungsarm 21 bzw. dem Referenzarm 23 in den zweiten Strahlteiler 24 ist ein erstes bzw. zweites Umlenkprisma 26 bzw. 28 vorgesehen.

Der erste, zweite und dritte Referenzspiegel 11, 12 bzw. 25 müssen keine Spiegel im engeren Sinne sein, sondern sind allgemein als Flächen aufzufassen, welche das im ersten bzw. zweiten Interferometer 10 bzw. 20 befindliche Licht zumindest teilweise reflektieren, weshalb der erste, zweite bzw. dritte Referenzspiegel 11, 12 bzw. 25 auch als erster, zweiter bzw. dritter Reflektor bezeichnet werden kann.

Die im zweiten Strahlteiler 24 überlagerten Teilstrahlen gelangen über den Probenarm 22 des zweiten Interferometers 20 in die Probe 1, werden dort an Grenzflächen zwischen Medien mit unterschiedlichen Brechungsindizes, z.B. Membranen oder Zellschichten, reflektiert und gelangen schließlich über den Probenarm 22 und den zweiten Strahlteiler 24 in den Ausgangsarm 27, von dem aus sie in einen zweiten Lichtleiter 29 eingekoppelt und über diesen einem Detektorobjektiv 31 zugeführt werden, welches das durch den Lichtleiter 29 geleitete Licht auf die Fläche eines zweidimensionalen Detektors 30 vergrößernd abbildet.

Der Detektor 30 ist vorzugsweise ein Halbleiterdetektor in CMOS-Technologie und weist eine Vielzahl von in einer Fläche angeordneten Detektorelementen (Pixel) auf, typischerweise 640 x 512 Pixel. Aufgrund der hierdurch möglichen zeitgleichen ("parallelen") Erfassung einer Vielzahl von Reflexionen in unterschiedlichen lateralen Positionen aus einer Ebene in einer bestimmten Tiefe der Probe 1 kann diese Art der OCT auch als "parallele OCT" bezeichnet werden.

Die bei der Erfassung des auf die einzelnen Detektorelemente des Detektors 30 auftreffenden Lichts erzeugten Detektorsignale werden in einer elektrischen Schaltung 32 weiterverarbeitet und schließlich an ein Computersystem 16 zur graphischen Darstellung und ggf. Bearbeitung weitergeleitet.

Gegenüber OCT-Systemen mit nur einem Interferometer sind bei dem hier beschriebenen OCT-System die Bewegung des zweiten Referenzspiegels 12 zur spektralen Modulation des eingekoppelten Lichts 14, die unmittelbare Erfassung des von der Probe 1 reflektieren Lichts und die Bilderfassung auf drei räumlich getrennte Komponenten verteilt, nämlich auf das erste Interferometer 10, das zweite Interferometer 20, das den Messkopf darstellt, bzw. den Detektor 30.

Durch die Verlagerung der Bewegung des zweiten Referenzspiegels 12 und der Bilderfassung auf separate Komponenten kann das zweite Interferometer 20, und damit der Messkopf, sehr kompakt und leicht handhabbar ausgestaltet werden. Dies macht das vorliegende OCT-System für Anwendungen an schwer zugänglichen äußeren oder inneren Stellen eines zu untersuchenden Körpers besonders geeignet.

In den folgenden Abschnitten werden bevorzugte Ausgestaltungen des erfindungsgemäßen Systems sowie vorteilhafte Kombinationen einzelner Ausgestaltungen näher beschrieben.

## 1. Tiefenscan durch makroskopische Bewegung des Referenzspiegels

Der bewegliche zweite Referenzspiegel 12 im ersten Interferometer 10 weist einen optischen Abstand I zum ersten Strahlteiler 13 auf und führt, ausgehend von einer Ausgangsposition N, eine lineare, vorzugsweise periodische, Bewegung in Richtung auf den ersten Strahlteiler 13 bzw. vom ersten Strahlteiler 13 weg mit einer optischen Weglänge L bzw. Amplitude A aus, wobei die optische Weglänge L bzw. die Amplitude A mindestens 100 mal, vorzugsweise 1000 mal, größer ist als die mittlere Wellenlänge $\lambda_0$ des in das erste Interferometer 10 eingekoppelten Lichts 14.

Der optische Abstand I ist hierbei gegeben durch das Produkt aus dem räumlichen Abstand des zweiten Referenzspiegels 12 zum ersten Strahlteiler 13 und dem Brechungsindex des zwischen dem zweiten Referenzspiegel 12 und dem ersten Strahlteiler 13 befindlichen Mediums.

Bei der hier dargestellten bevorzugten Ausgestaltung des ersten Interferometers 13 als sog. Freistrahl-Interferometer, bei welchem sich zwischen dem zweiten Referenzspiegel 12 und dem ersten Strahlteiler 13 Luft oder Vakuum befindet und der Brechungsindex etwa gleich 1 ist, ist der optische Abstand I des zweiten Referenzspiegels 12 sowie der optische Weg L, um welchen der optische Abstand I verändert wird, identisch mit dessen räumlichem Abstand bzw. räumlichem Weg. Die makroskopische Veränderung des optischen Abstands des zweiten Referenzspiegels 12 wird in diesem Fall durch eine makroskopische Bewegung des zweiten Referenzspiegels 12 um einen räumlichen Weg realisiert, welcher wesentlich größer ist als die mittlere Weglänge $\lambda_0$ des in

das erste Interferometer eingekoppelten Lichts 14.

Alternativ kann bei einer Ausgestaltung des ersten Interferometers 10 als sog. Faser-Interferometer (nicht dargestellt) zwischen dem zweiten Referenzspiegel 12 und dem ersten Strahlteiler 13 ein lichtleitendes Element, insbesondere eine Lichtleitfaser, vorgesehen sein, dessen optische Länge gezielt um einen optischen Weg veränderbar ist. Solche Lichtleitfasern werden auch als Fiber Stretcher bezeichnet. In diesem Fall ist der optische Abstand sowie der optische Weg, um welchen der optische Abstand verändert wird, durch das Produkt aus dem räumlichen Abstand bzw. dem räumlichen Weg, um welchen der Abstand verändert wird, und dem Brechungsindex des Lichtleitelements, welcher typischerweise im Bereich um 1,5 liegt, gegeben.

Die mittlere Wellenlänge $\lambda_0$ des in das erste Interferometer 10 eingekoppelten Lichts 14 liegt typischerweise im infraroten Spektralbereich, vorzugsweise zwischen 750 und 1350 nm.

Im Falle einer breitbandigen Lichtquelle 15 liegt die mittlere Wellenlänge $\lambda_0$ des Lichts 14 vorzugsweise in einem spektralen Bereich, in welchem das Licht 14 der Lichtquelle 15 ein Intensitätsmaximum aufweist. Alternativ dazu ist die mittlere Wellenlänge $\lambda_0$ durch einen Mittelwert aus allen von der Lichtquelle 15 emittierten Wellenlängen gegeben.

Vorzugsweise liegt die mittlere Wellenlänge $\lambda_0$ des in das erste Interferometer 10 eingekoppelten Lichts 14 in einem Wellenlängenbereich, in welchem der Detektor 30 eine sehr hohe, insbesondere die höchste, Empfindlichkeit aufweist. Im dargestellten System hat das Licht 14 eine mittlere Wellenlänge $\lambda_0$ von etwa 1300 nm und eine Halbwertsbreite (FWHM) von etwa 200 nm.

Bei einer mittleren Wellenlänge $\lambda_0$ des Lichts 14 im Bereich von z.B. 1 $\mu$m liegt die optische Weglänge L bzw. Amplitude A der Bewegung des Referenzspiegels 12 also mindestens bei etwa 0,1 mm, vorzugsweise mindestens bei etwa 1 mm.

Im Gegensatz zu der im Stand der Technik üblichen mikroskopischen Amplitude der Referenzspiegelbewegung in der Größenordnung von Bruchteilen der mittleren Wellenlänge $\lambda_0$ des eingekoppelten Lichts 14, also von bis zu typischerweise 1 $\mu$m, erfolgt im beschriebenen System eine makroskopische Bewegung des zweiten Referenzspiegels 12 in der Größenordnung von 0,1 mm bis zu mehreren Millimetern.

Während der makroskopischen Linearbewegung des zweiten Referenzspiegels 12 wird das von der Probe 1 reflektierte Licht über das zweite Interferometer 20, den zweiten Lichtleiter 29 und die Detektoroptik 31 zum zweidimensionalen Detektor 30 weitergeleitet und von diesem sukzessive zu mehreren Zeitpunkten für jeweils eine bestimmte Zeitdauer, welche der Integrationszeit des Detektors 30 entspricht, erfasst und in entsprechende Detektorsignale umgewandelt.

Damit eine Interferenz zwischen dem vom dritten Referenzspiegel 25 und dem von der Probe 1 reflektierten Licht auftreten kann, muss die sog. Kohärenzbedingung erfüllt sein, welche u.a. besagt, dass die jeweils reflektierten Lichtwellen eine konstante Phasenbeziehung zueinander haben müssen, um miteinander interferieren zu können. Aufgrund der Verwendung von Licht 14 mit einer sehr kurzen Kohärenzlänge von typischerweise 10 $\mu$m ist die Bedingung einer konstanten Phasenbeziehung nur in bestimmten Tiefen oder Tiefenbereichen der Probe 1 erfüllt, welche daher auch als Kohärenz-Gate bezeichnet werden.

Jede Position des zweiten Referenzspiegels 12 während der makroskopischen Bewegung entspricht dabei einer bestimmten Tiefe innerhalb der Probe 1 oder einem Tiefenbereich um diese bestimmte Tiefe herum, für welche bzw. welchen die Kohärenzbedingung erfüllt ist, so dass eine Interferenz zwischen dem vom dritten Referenzspiegel 25 und dem von der Probe 1 reflektierten Licht auftreten kann.

Im Falle einer periodischen Bewegung des zweiten Referenzspiegels 12 können beide Halbperioden der periodischen Bewegung des zweiten Referenzspiegels 12 jeweils zur Aufnahme von Detektorsignalen genutzt werden.

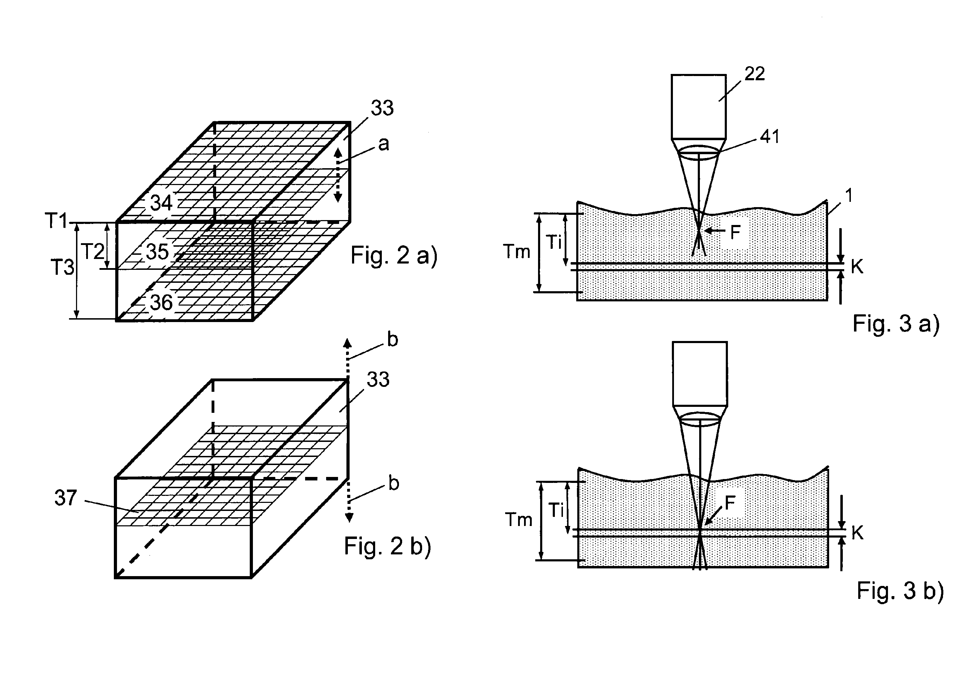

Auf diese Weise werden durch den Detektor 30 sukzessive zweidimensionale Schnitte aus unterschiedlichen Tiefen der Probe 1 aufgenommen. Dies ist in Fig. 2 a) veranschaulicht, in welcher - stellvertretend für eine Vielzahl von zweidimensionalen Schnitten - ein erster, zweiter und dritter zweidimensionaler Schnitt 34, 35 bzw. 36 durch ein Raumelement 33 der Probe 1 dargestellt ist. Ein solcher zweidimensionaler Schnitt "wandert" synchron mit der makroskopischen Bewegung des zweiten Referenzspiegels 12 in Richtung a durch das betrachtete Raumelement 33 der Probe 1, ohne dass diese selbst bewegt werden muss.

Jeder Schnitt 34, 35 bzw. 36 liegt in einer Tiefe T1, T2 bzw. T3 der Probe 1, in welcher jeweils die Kohärenzbedingung erfüllt ist, so dass eine Interferenz zwischen dem vom dritten Referenzspiegel 25 und dem von der Probe 1 reflektierten Licht auftreten kann. Die makroskopische Bewegung des zweiten Referenzspiegels 12 in Kombination mit der sukzessiven zweidimensionalen Erfassung des von der Probe 1 reflektierten Lichts hat somit die Wirkung eines dreidimensionalen Tiefenscans.

Fig. 2 b) zeigt im Vergleich dazu ein im Stand der Technik eingesetztes Verfahren. Um unterschiedlich tiefe Schnitte 37 durch das betrachtete Raumelement 33 zu erhalten, muss die Probe 1 selbst in Richtung b relativ zum Interferometer bewegt werden, während die absolute Lage des Schnittes 38 im Raum im Wesentlichen unverändert bleibt.

Die oben beschriebene Kombination der makroskopischen Linearbewegung des Referenzspiegels 12 einerseits mit der Erfassung des von der Probe 1 reflektierten Lichts mit einem zweidimensionalen Detektor 30 andererseits ermöglicht dem gegenüber eine wesentlich einfacher zu realisierende und schnellere Aufnahme eines vollständigen dreidimensionalen Datensatzes des gewünschten Raumelements 33 der Probe 1. Durch die makroskopische Bewegung des zweiten Referenzspiegels 12 wird hierbei ein dreidimensionales Tomogramm anstatt eines nur zweidimensionalen Bildes aus einer bestimmten Tiefe erhalten. Im Gegensatz zu Systemen nach dem Stand der Technik braucht bei dieser Methode zur Aufnahme eines dreidimensionalen Datensatzes die Probe 1 relativ zum zweiten Interferometer 20 nicht mehr bewegt zu werden. Dies macht das beschriebene OCT-System kompakt, zuverlässig und einfach handhabbar, so dass dieses besonders für den Einsatz *in vivo* geeignet ist.

Der auf diese Weise erhaltene dreidimensionale Datensatz erlaubt eine exakte Diagnose, insbesondere bei biologischen Proben. Hierbei können softwaregestützte Diagnosehilfen mit besonders hoher Effizienz eingesetzt werden, wie z.B. das sog. "3d-Rendering", bei welchem ein dreidimensionaler Datensatz durch eine spezielle Software so bearbeitet wird, dass auf einem zweidimensionalen Monitor ein quasi dreidimensionales Bild erzeugt wird. Hierfür können beispielsweise Hohlräume oder Gewebeablösungen als dreidimensionale Animation - vergleichbar mit der Computer-Tomographie (CT) - dargestellt werden.

2. Fokusnachführung

Das oben beschriebene OCT-System ist so entworfen, dass während eines vollen Hubs, d.h. der Weglänge L bzw. der doppelten Amplitude A, der Bewegung des zweiten Referenzspiegels 12 stets ein Interferenzsignal mit ausreichend hoher Intensität und hoher Schärfe erhalten wird. Durch die nachfolgend näher beschriebene Fokusnachführung wird gewährleistet, dass das Interferenzsignal sowie die Schärfe des erfassten Interferenzmusters für alle Tiefen in der Probe 1 maximal sind.

Dazu wird während der Erfassung des von der Probe 1 reflektierten Lichts der Fokus, d.h. der Brennpunkt, der probenseitigen Abbildungsoptik des zweiten Interferometers 20 in der Weise eingestellt, dass die Lage des Fokus in der Probe 1 und die Lage derjenigen Ebene in der Probe 1, bei welcher im Falle einer Reflexion von Licht die Kohärenzbedingung erfüllt ist und Interferenz auftritt, zu allen Zeiten während der Aufnahme eines Tomogramms des Raumelements 33 der Probe 1 im Wesentlichen identisch sind. Dies wird nachfolgend anhand der Fig. 3 a) und 3 b) veranschaulicht.

Fig. 3 a) zeigt den Fall, bei welchem der Fokus F des - hier nur vereinfacht als Linse dargestellten - Probenobjektivs 41 des Probenarms 22 in einer Tiefe der Probe 1 liegt, die nicht mit der Lage des Kohärenz-Gates K übereinstimmt. Der innerhalb des Kohärenz-Gates K in der Tiefe Ti erfasste Schnitt durch die Probe 1 wird dadurch nicht exakt scharf auf den Detektor 30 (siehe Fig. 1) abgebildet, so dass Informationsverluste bei der Erfassung der Interferenz hinzunehmen wären. In Fig. 3 b) ist dagegen der Fall dargestellt, bei welchem der Fokus F des Probenobjektivs 41 derart eingestellt wurde, dass er innerhalb des Kohärenz-Gates K in der Tiefe Ti liegt. Dieses Nachführen des Fokus F des Probenobjektivs 41 entsprechend der jeweiligen Tiefe Ti des Kohärenz-Gates K wird als Fokus Tracking bezeichnet. Auf diese Weise wird das zweite Interferometer 20 während des Tiefenscans auf die jeweilige Lage des Kohärenz-Gates K in unterschiedlichen Tiefen Ti der Probe 1 scharf gestellt, so dass aus jeder Tiefe der Probe 1 Bilder mit hoher Schärfe erhalten werden.

Die maximale optische Scantiefe Tm gibt an, bis zu welcher Tiefe unterhalb der Oberfläche der Probe 1 die Kohärenzbedingung für eine konstruktive Interferenz erfüllt und entsprechende Interferenzmuster erhalten werden.

Durch die Fokusnachführung wird außerdem erreicht, dass in jeder abgetasteten Tiefe Ti in der Probe 1 die beleuchteten Flächen auf dem unbeweglichen dritten Referenzspiegel 25 im zweiten Interferometer 20 einerseits und in der jeweiligen Tiefe der Probe 1 andererseits identisch sind. Darüber hinaus sind die Abbildungen der jeweiligen beleuchteten Flächen über den Referenzarm 23 und den Probenarm 22 in der gemeinsamen Bildebene 27a von Referenz- und Probenarm 23 bzw. 22 identisch und exakt überlagert.

Nachfolgend werden bevorzugte Ausführungen des beschriebenen OCT-Systems zur Realisierung der Fokusnachführung näher erläutert.

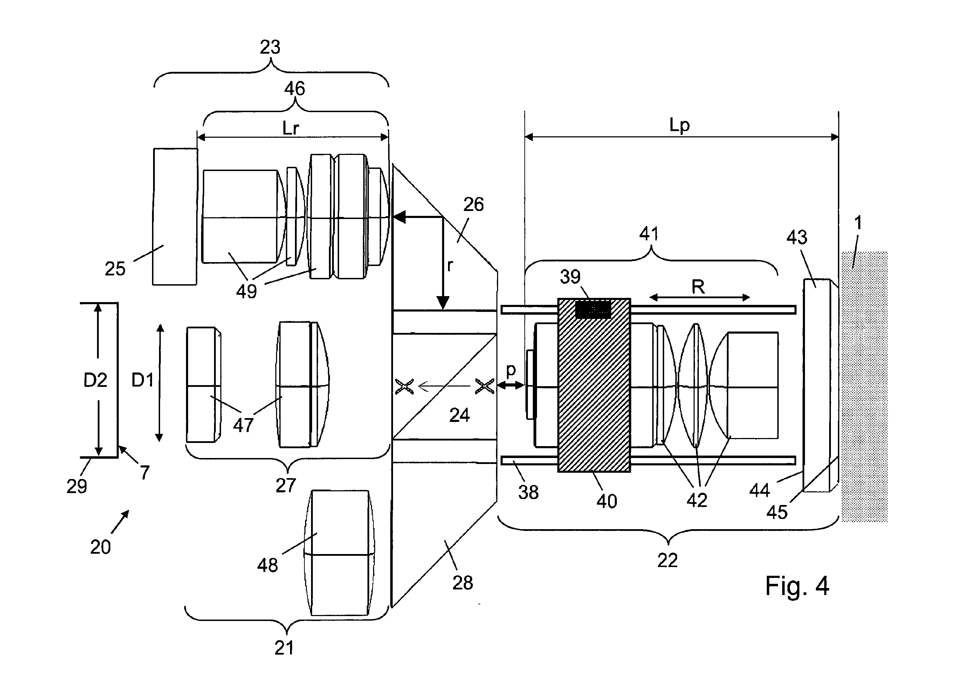

Fig. 4 zeigt einen Querschnitt durch die Anordnung der einzelnen optischen Komponenten im zweiten Interferometer 20. Das Probenobjektiv 41 im Probenarm 22 umfasst vorzugsweise mehrere Linsen 42, die einzeln und/oder gruppenweise in Richtung R auf die Probe 1 bzw. von dieser weg bewegt werden können. Hierzu ist ein piezoelektrischer Aktuator 40, insbesondere ein Ultraschall-Piezo-Motor, vorgesehen, der mit dem Probenobjektiv 41 bzw. den Linsen 42 gekoppelt ist und dieses bzw. diese entlang einer oder mehrer Führungen 38, insbesondere Führungsstäbe oder Führungsnuten, bewegt.

Die Bewegung der Linsen 42 erfolgt vorzugsweise synchron mit der makroskopischen Bewegung des Referenzspiegels 12 im ersten Interferometer 10 (siehe Fig. 1). Auf diese Weise folgt der Fokus F des Probenobjektivs 41 dem Kohärenz-Gate G, während Letzteres sukzessive unterschiedliche Tiefen T1, T2 bzw. T3 der Probe 1 durchfährt, aus denen mit Hilfe des Detektors 30 jeweils zweidimensionale Schnitte 34, 35 bzw. 36 (vgl. Fig. 2) aufgenommen werden.

Die Synchronisation der makroskopischen Bewegung des Referenzspiegels 12 und der Fokusnachführung einerseits in Kombination mit einem zweidimensionalen Detektor 30 andererseits gewährleistet eine besonders einfache und schnelle Aufnahme einer Vielzahl von scharfen zweidimensionalen Bildschnitten in unterschiedlichen Tiefen der Probe 1 und damit die Erfassung eines vollen dreidimensionalen Bilddatensatzes mit hoher Bildqualität.

Da das erste Interferometer 10 und die optische Abbildung im Probenarm 22 kontinuierlich aufeinander abgestimmt werden, sind die vom Detektor 30 erfassten Interferenzsignale für jede Tiefe in der Probe 1 maximal, so dass sich ein sehr hohes Signal-zu-Rausch-Verhältnis ergibt. Darüber hinaus wird dadurch sicher gestellt, dass die laterale Auflösung für alle Tiefen in der Probe 1 optimal ist, da der Fokus F der Abbildung stets im Kohärenz-Gate K liegt. Es werden dadurch detailgetreue OCT-Bilder mit hohem Kontrast erhalten.

Vorteilhafterweise ist die Geschwindigkeit v2 der Bewegung der Linsen 42 des Probenobjektivs 41 in Richtung R kleiner als die Geschwindigkeit v1 der Bewegung des Referenzspiegels 12. Vorzugsweise wird hierbei ein Verhältnis v1/v2 der Geschwindigkeiten des Referenzspiegels 12 und der Linsen 42 gewählt, das näherungsweise gleich 2 n - 1 ist oder bis zu etwa $\pm$ 20 %, vorzugsweise bis zu etwa $\pm$ 10 %, um diesen Wert liegt. Hierdurch werden die Lage des Fokus F und Kohärenz-Gates G mit besonders hoher Zuverlässigkeit aufeinander abgestimmt, wie durch nachfolgende Überlegung erklärt werden kann.

Der Fokus F des Probenobjektivs 41 liegt in einer Probe 1, deren Brechungsindex n im Allgemeinen ungleich eins ist. Verschiebt man einerseits das Probenobjektiv 41 um einen bestimmten Weg in Richtung R der Probe 1, so verschiebt sich der Fokus F in der Probe um einen bestimmten Betrag $d_F$. Beispielsweise führt die Verschiebung des Probenobjektivs 41 um 0.78 mm bei einem Brechungsindex der Probe 1 von 1,4 zur Verschiebung des Fokus in der Probe 1 um etwa $d_F$ = 1 mm. Wird andererseits der Referenzspiegel 12 um einen bestimmten Weg verschoben, so verschiebt sich das Kohärenz-Gate K ebenfalls um einen bestimmten Betrag $d_K$. Beispielsweise ergibt eine Verschiebung des Referenzspiegels 12 um 1.4 mm bei einem Brechungsindex n = 1,4 eine Verschiebung des Kohärenz-Gates K um etwa $d_K$ = 1 mm. Hierdurch würden das Kohärenz-Gate K und der Fokus F bei einer Verschiebung des Referenzspiegels 12 und des Probenobjektivs 41 jeweils um denselben Weg bei einem Tiefenscan über einen makroskopischen Tiefenbereich auseinander laufen.

Durch die oben beschriebene Wahl des Verhältnisses v1/v2 der Geschwindigkeiten des Referenzspiegels 12 und der Linsen 42 wird gewährleistet, dass das Kohärenz-Gate K und der Fokus F während des Tiefenscans im gesamten betrachteten Tiefenbereich übereinander liegen. Im obigen Beispiel einer Probe mit einem Brechungsindex n = 1,4 liegt das Verhältnis v1/v2 der Geschwindigkeiten im Bereich von etwa (2-1,4 - 1) $\pm$ 20 %, d.h. zwischen etwa 1,44 und 2,16, und beträgt vorzugsweise etwa 2·1,4 - 1 = 1,8.

Die Synchronisation der Bewegung des Referenzspiegels 12 und der Linsen 42 erfolgt bevorzugt in der Weise, dass Referenzspiegel 12 und Linsen 42 zu einem bestimmten Zeitpunkt zwei unterschiedliche, vordefinierte räumliche Punkte mit jeweils konstanten, vordefinierten und unterschiedlichen Geschwindigkeiten v1 bzw. v2 durchlaufen.

Nach dem Durchlaufen der räumlichen Punkte beginnt die Aufnahme der eigentlichen OCT-Signale bis zur vordefinierten Tiefe in der Probe 1. Bei einer periodischen Vorwärts- und Rückwärtsbewegung des Referenzspiegels 12 können hierbei sowohl während der Vorwärts- als auch während der Rückwärtsbewegung des Referenzspiegels 12 OCT-Signale aufgenommen werden. Die Synchronisation von Referenzspiegel 12 und Linsen 42 erfolgt hierbei analog und wird nach jedem Richtungswechsel neu eingestellt.

Der Messkopf, in dem sich das Probenobjektiv 41 befindet, ist relativ zum ersten Interferometer 10, in welchem sich der zweite Referenzspiegel 12 befindet, frei beweglich. Eine mechanische Kopplung von Probenobjektiv 41 und Referenzspiegel 12 zur Synchronisation der Linsen- und Referenzspiegelbewegungen würde zu einer unzureichenden Genauigkeit der Synchronisation führen.

Die Synchronisation der Bewegungen des Referenzspiegels 12 einerseits und der Linsen 42 des Probenobjektivs 41 andererseits erfolgt daher vorzugsweise auf elektronischem Wege. Dabei ist es vorteilhaft, im Bereich des Referenzspiegels 12 und der Linsen 42 des Probenobjektivs 41 jeweils einen Positionssensor 5 bzw. 39 vorzusehen, welcher die aktuelle Referenzspiegel- bzw. Linsenposition erfasst und in entsprechende Positionssignale umwandelt. Die beiden Positionssignale werden einer Steuerungseinheit, insbesondere dem Computersystem 16, zugeführt, welche darauf hin den Antrieb des Referenzspiegels 12 bzw. der Linsen 42 entsprechend steuert.

Die Steuerung des Referenzspiegels 12 bzw. der Linsen 42 erfolgt vorzugsweise über eine Rückkopplung der Positionssignale mittels eines sog. Master-Slave-Systems. Bei einem solchen Master-Slave-System ist ein gemessener Positionswert in einer ersten Positioniereinheit die Grundlage für einen Soll-Wert des Regelkreises für eine zweite Positioniereinheit. Im vorliegenden Fall wird die gemessene Position der ersten Positioniereinheit des Referenzspiegels 12 mit einem Faktor kleiner 1 multipliziert und der zweiten Positioniereinheit der Linsen 42 als neuer Soll-Wert zugeführt. Der relative Positionsfehler zwischen dem beweglichen Referenzspiegel 12 und den Linsen 42 wird dadurch minimiert, selbst bei einem relativ großen absoluten Positionierfehler der ersten Positioniereinheit. Die beiden Komponenten sind dadurch auf elektronische Weise wie über ein mechanisches Getriebe miteinander verkoppelt, wodurch dies auch als Electronic Gearing bezeichnet werden kann.

Die Fokusnachführung kann alternativ oder zusätzlich dadurch realisiert werden, dass im Probenobjektiv 41 eine adaptive Linse vorgesehen ist, deren Abbildungseigenschaften sich gezielt steuern und verändern lassen. Beispielsweise kann eine Öl-Wasser-Linse elektrisch derart angesteuert werden, dass sich deren Krümmungsradien verändern, wodurch deren Fokus verändert und auf einfache Weise an die jeweilige Lage des Kohärenz-Gates angepasst werden kann. In diesem Fall muss die Geschwindigkeit und der Beginn der Änderung des Fokus F der adaptiven Linse mit der Bewegung des Referenzspiegels 12 analog zu den oben beschriebenen Verfahren synchronisiert werden.

### 3. Automatische Kalibrierung der Fokusnachführung

Am probenseitigen Ende des Probenarms 22 des als Messkopf ausgebildeten zweiten Interferometers 20 ist eine Materialschicht 43 (siehe Fig. 4) versehen, welche vorzugsweise aus Saphirglas besteht. Die Materialschicht 43 ist auf der Innenseite 44 mit einer Anti-Reflex-Schicht beschichtet und auf der probenseitigen Außenseite 45 vorzugsweise unbeschichtet.

Das OCT-System kann in einem Diagnosemodus und in einem Kalibriermodus betrieben werden. Im Diagnosemodus, der dem normalen Messbetrieb entspricht, wird die probenseitige Außenseite 45 der Materialschicht 43 mit einem sog. Index Matching-Gel bestrichen und in Kontakt mit der zu untersuchenden Probe 1 gebracht, von welcher dreidimensionale Bilder aufgenommen werden. Im Kalibriermodus wird die relative Lage des Fokus F des Probenobjektivs 41 zum Kohärenz-Gate K bestimmt, wobei die Außenseite 45 der Materialschicht 43, welche sich während des Kalibrierverfahrens vorzugsweise in Luft befindet, als Referenzfläche dient.

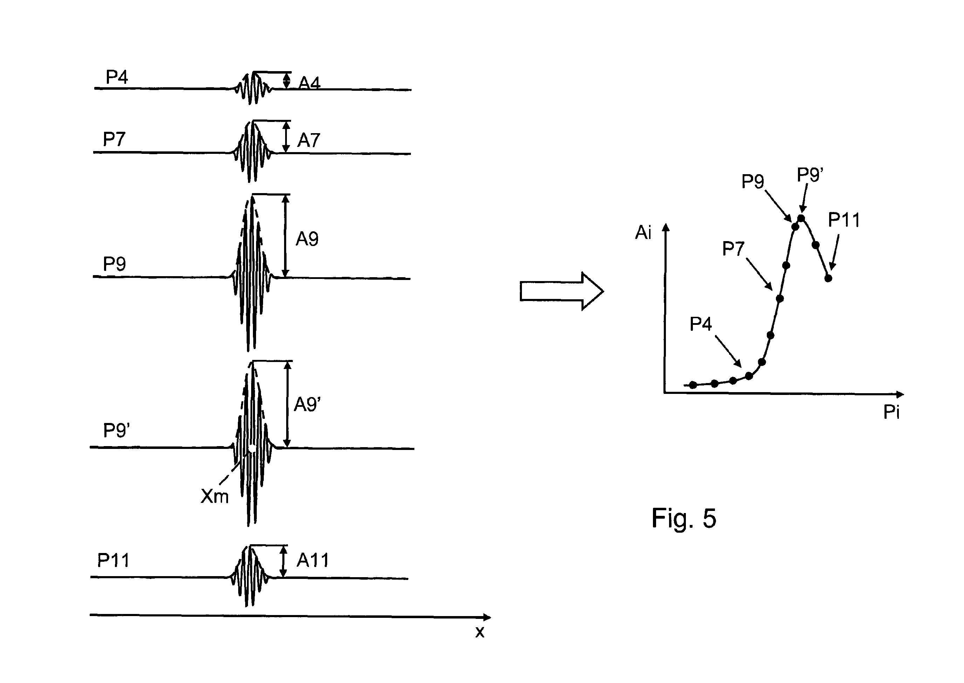

Im Kalibriermodus wird die Amplitude des OCT-Signals, das von einer Reflexion des Lichts aufgrund des Übergangs des Lichts von der Materialschicht 43 in Luft verursacht wird, für verschiedene Positionen des Probenobjektivs 41 gemessen, wobei folgende Verfahrensschritte durchgeführt werden, die anhand der Figuren 4 und 5 veranschaulicht werden:

　　a) die Gruppe der Linsen 42 wird in eine Ausgangsposition gebracht, indem sie so nahe wie möglich an den zweiten Strahlteiler 24 gefahren wird;
　　b) die Gruppe der Linsen 42 wird in dieser Position belassen;
　　c) während einer makroskopischen Bewegung des zweiten Referenzspiegels 12 wird die Amplitude Ai des Maximums des Interferenz-Signals bestimmt;
　　d) die Gruppe der Linsen 42 wird um wenige Mikrometer, typischerweise 5 bis 20 $\mu$m, vom zweiten Strahlteiler 24 fortbewegt und in dieser Position gehalten;
　　e) die Schritte c) bis d) werden für mehrere unterschiedliche Positionen P1 bis P11 der Linsen 42 wiederholt, wobei für jede Position P1 bis P11 der Linsen 42 eine Amplitude A1 bis A11 des Maximums des jeweiligen Interferenz-Signals erhalten wird;
　　f) es wird diejenige Position P9 der Gruppe der Linsen 42 ermittelt, an welcher die Amplitude A9 am größten ist;
　　g) die Schritte c) bis f) werden in der Nähe der Position P9 dieses Maximums mit kleinerer Schrittweite, typischerweise 0,5 $\mu$m bis 5 $\mu$m, wiederholt, wobei diejenige Position P9' der Gruppe der Linsen 42 ermittelt wird, an welcher die Amplitude A9' am größten ist;
　　h) aus der dieser Position P9' der Gruppe der Linsen 42 zugeordneten Referenzspiegelbewegung wird die Position Xm des beweglichen Referenzspiegels 12 ermittelt, bei der das Interferenz-Signal maximal ist.

Die Kalibrierung kann alternativ auch in der Weise durchgeführt werden, dass sich das Probenobjektiv 41 während der Kalibrierung auf den zweiten Strahlteiler 24 zu bewegt.

Befinden sich die Gruppe der Linsen 42 in der Position P9' und der Referenzspiegel 12 in der Position Xm, so sind Kohärenz-Gate und Fokuslage identisch. Die ermittelten Positionen P9' bzw. Xm werden im Diagnosemodus als Anfangsposition der Linse bzw. Linsen bzw. des Reflektors eingestellt.

Auf diese Weise werden Änderungen im OCT-System automatisch korrigiert, ohne dass hierfür eine zusätzliche Hardware benötigt wird. Selbst wenn die Materialschicht verschmutzt oder mit Index Matching-Gel bestrichen sein sollte, würde das beschriebene Verfahren funktionieren, da dann der Übergang des Lichts von Schmutz zu Luft bzw. Gel zu Luft genutzt würde. Das Verfahren ist sehr schnell und dauert nur wenige Sekunden. Es kann dadurch häufig durchgeführt werden, wodurch eine hohe Zuverlässigkeit des Systems gewährleistet wird.

Um die Genauigkeit des beschriebenen Kalibrierverfahrens noch weiter zu erhöhen, kann ein zusätzliches Element aus Glas oder Kunststoff auf die Materialschicht aufgesetzt werden, ein sog. Target. Das oben beschriebene Verfahren wird dann für zwei oder mehrere Tiefen innerhalb des zusätzlichen Elements durchgeführt. Dadurch kann nicht nur ein Offset, d.h. eine Versetzung der Bezugspunkte der Bewegung des Referenzspiegels 12 und der Linsen 42, sondern auch eine etwaige Nichtlinearität korrigiert werden. Bei dem oben beschriebenen Kalibrierverfahren werden dann mehrere Referenzflächen genutzt, wobei mehrere Positions-Paare bestimmt werden, für welche Fokuslage und Kohärenz-Gate identisch sind. Dadurch kann nicht nur ein konstanter relativer Positionsfehler zwischen den beiden Positionier-Einheiten korrigiert werden, sondern es können auch etwaige Fehler in der relativen Linearität oder der relativen Geschwindigkeit der beiden Einheiten korrigiert werden. Solche Fehler können sich z.B. durch Alterung der Positionssensoren 5 bzw. 39 ergeben, wenn sich beispielsweise die Positions-Empfindlichkeit eines der beiden Positionssensoren 5 bzw. 39 ändert.

Zusammenfassend ist festzuhalten, dass die dynamische Synchronisation von Fokus-Lage und Kohärenz-Gate im Diagnosemodus des beschriebenen OCT-Systems zu einer Vielzahl von Vorteilen im Hinblick auf Bildqualität und Zuverlässigkeit führt. Bei zusätzlicher, insbesondere regelmäßiger, Anwendung des beschriebenen Kalibriermodus kann diese Synchronisation über einen langen Zeitraum garantiert werden.

4. Modulation der Intensität der Lichtquelle

Bei dem beschriebenen OCT-System wird das entstehende Interferenzmuster mit dem Detektor 30 erfasst, wobei ein entsprechendes Interferenzsignal erzeugt wird. Die Abtastrate des Detektors 30 zum Abtasten des Interferenzsignals muss dabei so gewählt werden, dass die zeitliche Variation der Interferenzstruktur mit ausreichender Genauigkeit erfasst werden kann. Dies erfordert im Allgemeinen hohe Abtastraten, wenn hohe Geschwindigkeiten für einen Tiefenscan erzielt werden sollen.

Da die einzelnen Perioden einer Interferenzstruktur im Allgemeinen jeweils zu mehreren Zeitpunkten abgetastet werden müssen, ist die maximal mögliche Scan-Geschwindigkeit in Richtung der Tiefe der Probe 1 abhängig von der maximal möglichen Abtastrate des Detektors 30. Bei Verwendung von schnellen Detektor-Arrays mit hoher räumlicher Auflösung, d.h. großer Anzahl von Detektorelementen pro Längeneinheit, liegt die maximale Abtastrate typischerweise im Bereich von etwa 1 kHz. Dies führt bei einer mittleren Wellenlänge des eingekoppelten Lichts 14 von beispielsweise 850 nm zu einer maximalen Geschwindigkeit für den Tiefenscan von etwa 0,1 mm/s, wenn vier Punkte pro Periode der Interferenzstruktur aufgenommen werden.

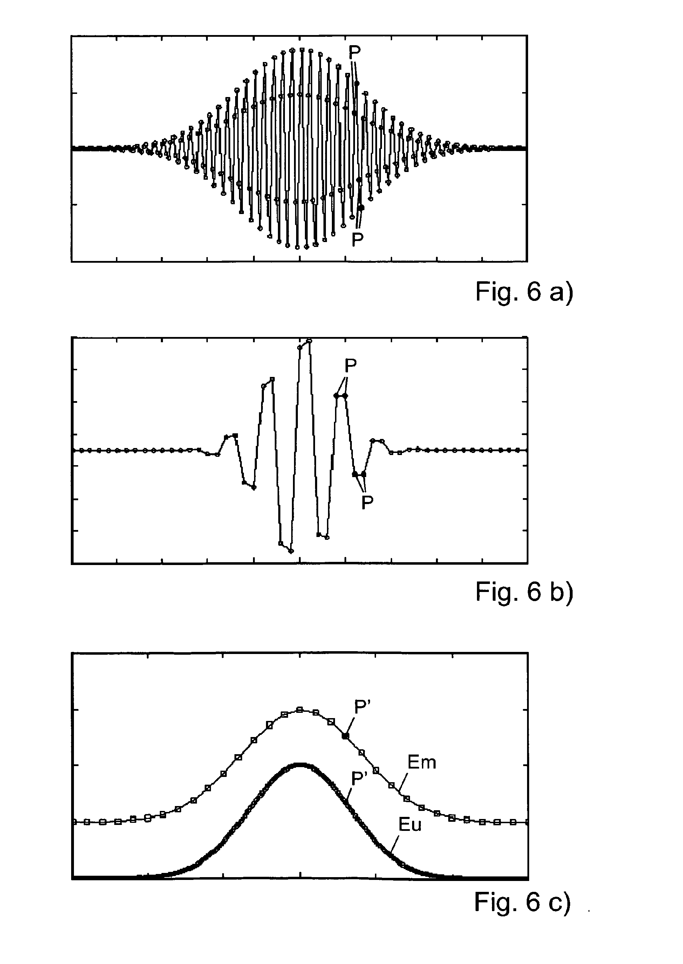

Fig. 6 a) zeigt den zeitlichen Verlauf eines typischen Interferenzsignals, das mit einer Abtastrate von jeweils vier Abtastzeitpunkten P je Periode abgetastet wird. In der Figur sind beispielhaft vier solcher Punkte innerhalb einer Periode des Interferenzsignals eingezeichnet.

Zur Erhöhung der Geschwindigkeit des Tiefenscans wird im vorliegenden OCT-System die Intensität des in das erste Interferometer 10 eingekoppelten Lichts 14 zeitlich moduliert. Diese Modulation erfolgt periodisch, wobei deren Frequenz um einen bestimmten Betrag, vorzugsweise um bis zu 40 %, größer oder kleiner ist als die Dopplerfrequenz $f_D$, welche durch die mittlere Wellenlänge $\lambda_0$ des eingekoppelten Lichts 14 und die Geschwindigkeit v des beweglichen Referenzspiegels 12 gegeben ist: $f_D = 2v/\lambda_0$. Typische Frequenzen dieser Modulation liegen im Bereich zwischen 1 kHz und 25 kHz.

Alternativ oder zusätzlich kann auch die Intensität des vom ersten Interferometer 10 ausgegebenen Lichts des dritten Teilstrahls 4 mit der Modulationsfrequenz $f_M$ moduliert werden, um die vorstehend beschriebene vorteilhafte Wirkung zu erzielen. Die Modulation erfolgt hierbei vorzugsweise während der Einkopplung des Lichts des dritten Teilstrahls 4 in den ersten Lichtleiter 17 am Ausgang 8 des ersten Interferometers 10. Die Intensitätsmodulation kann aber auch im zweiten Interferometer 10 vor der Ausgabe des Lichts des dritten Teilstrahls 4 erfolgen. Zur Modulation der Intensität des vom zweiten Interferometer 10 ausgegebenen Lichts ist vorzugsweise ein optisches Element vorgesehen, welches z.B. im ersten Interferometer 10 oder im Bereich des Ausgangs 8 des ersten Interferometers 10 angeordnet und in seinen Transmissions- oder Abbildungseigenschaften gezielt verändert werden kann. So kann beispielsweise durch ein adaptives optisches Element im Bereich des Ausgangs 8 des ersten Interferometers 10 die Intensität des vom ersten Interferometer 10 ausgegebenen Lichts des dritten Teilstrahls 4 periodisch von "hoch" auf "niedrig" geschaltet werden. Das optische Element kann aber auch im Strahlengang des ersten Interferometers 10, z.B. zwischen einem der Referenzspiegel 11 bzw. 12 und dem ersten Strahlteiler 13, angeordnet werden.

Die genaue Wahl der Modulationsfrequenz wird in Abhängigkeit von der mittleren Wellenlänge $\lambda_0$ des eingekoppelten Lichts 14 der Lichtquelle 15, der gewünschten Scan-Geschwindigkeit des Tiefenscans und der maximalen Abtastrate des Detektors 30 getroffen.

Bevorzugt wird die Modulationsfrequenz so gewählt, dass sie der maximalen Abtastrate des Detektors 30 oder einem ganzzahligen Vielfachen hiervon entspricht. Die maximale Abtastrate ist hierbei durch den reziproken Wert der minimalen Framezeit des Detektors 30 gegeben. Die minimale Framezeit des Detektors 30 setzt sich zusammen aus der Zeit, die mindestens notwendig ist, um ein vollständiges Bild aufzunehmen, und der minimalen Totzeit des Detektors 30, die verstreicht, bis das nächste Bild aufgezeichnet werden kann. Die minimale Framezeit nimmt im Allgemeinen mit zunehmender Größe des aufgezeichneten Bildes zu.

Die Form der Modulation der Intensität der Lichts 14 ist vorzugsweise sinus- oder rechteckförmig. Letztere Form kann z.B. einfach über ein rotierendes Chopperrad 18 (siehe Fig. 1) realisiert werden. Andere Möglichkeiten sind akusto-optische oder elektro-optische Modulatoren oder Flüssigkristall-Modulatoren. Auch eine direkte Modulation der Lichtquelle 15 ist möglich, indem diese so angesteuert wird, dass sie das Licht 14 mit zeitlich modulierter Intensität ausgibt.

Ein entsprechender Effekt kann alternativ oder zusätzlich dadurch erzielt werden, dass ein optisches Element, das z.B. vor oder nach dem ersten Strahlteiler 13 (siehe Fig. 1) angeordnet ist, in seiner Transmissions- oder Abbildungseigenschaft geschaltet wird. So könnte beispielsweise durch ein entsprechendes Schalten eines adaptiven optischen Elements die Einkoppeleffizienz des dritten Teilstrahls 4 in den ersten Lichtleiter 17 periodisch von "hoch" auf "niedrig" geschaltet werden.

Die beschriebene Modulation der Intensität des eingekoppelten Lichts 14 mit, vorzugsweise geringfügig, von der Dopplerfrequenz abweichender Modulationsfrequenz erzeugt eine niederfrequente Schwebung zwischen der Modulation und dem Interferenzsignal.

Fig. 6 b) zeigt den zeitlichen Verlauf eines aufgrund der beschriebenen Modulation des eingekoppelten Lichts 14 erhaltenen Schwebungssignals, welches - wie das Interferenzsignal im Beispiel der Fig. 6 a) - mit einer Abtastrate von jeweils vier Abtastzeitpunkten P je Periode abgetastet wird. Bei der Abtastung des Schwebungssignals sind aufgrund dessen geringerer Frequenz deutlich weniger Abtastzeitpunkte P pro Zeiteinheit erforderlich als bei der Abtastung des Interferenzsignals in Fig. 6 a), so dass bei einer festen, durch die Wahl des Detektors 30 gegebenen Abtastrate deutlich höhere Geschwindigkeiten für den Tiefenscan erreicht werden können.

Ein weiterer Vorteil dieses Verfahrens wird im Folgenden näher erläutert.

Die Integrationszeit des Detektors 30 entspricht der Zeitdauer, während welcher der Detektor 30 das im Bereich eines Zeitpunkts P auf die Detektorelemente treffende Licht erfasst und dabei integriert. Der Detektor 30 wird vorzugsweise so betrieben, dass die Integrationszeit nur unwesentlich kürzer ist als die Framezeit. Die Framezeit ist hierbei so gewählt, dass sie genau der Dauer einer Periode der Modulation oder einem ganzzahligen Vielfachen davon entspricht. Das in Fig. 6 b) gezeigte Schwebungssignal wurde durch Integration über die Dauer von zwei Perioden der Modulation erhalten.

Würde man die Scan-Geschwindigkeit ohne die vorstehend beschriebene Modulation der Intensität des Lichts 14 steigern, so müsste die Framezeit - und damit die Integrationszeit - des Detektors 30 kürzer werden, da die Dopplerfrequenz zunehmen würde und dadurch zeitlich enger liegende Abtastzeitpunkte P notwendig wären. Eine kürzere Integrationszeit würde jedoch zu einer Verringerung der pro Integration und pro Detektorelement gesammelten Photonen führen, was aufgrund des aus der statistischen Natur der Photonen resultierenden sog. Schott-Noise zu einer Verringerung des Signal-Rausch-Verhältnisses führen würde. Um das Signal-Rausch-Verhältnis wieder zu verbessern, müsste die Intensität des eingekoppelten Lichts 14 proportional zur Scan-Geschwindigkeit erhöht werden.

Steigert man dagegen die Scan-Geschwindigkeit mit Hilfe der oben beschriebenen Modulation der Intensität des Lichts 14, so kann die Integrationszeit konstant bleiben. Es ergibt sich lediglich ein Lichtverlust von 50 % aufgrund der Modulation des Lichts 14. Bei der bevorzugten Modulationsfrequenz, die dem doppelten reziproken Wert einer Framezeit entspricht, ergibt sich eine Steigerung der Geschwindigkeit um den Faktor 8. In diesem Fall ist viermal weniger Lichtintensität notwendig, um diese Geschwindigkeitssteigerung zu erreichen, als im Fall ohne Modulation. Die Auswirkungen des Lichtverlustes in Höhe von 50 % aufgrund der Modulation werden dadurch überkompensiert.

Die erforderliche Intensität des Lichts 14 der Lichtquelle 15 muss bei dem beschriebenen Verfahren - im Gegensatz zur direkten Abtastung ohne Schwebung - daher nicht mit der Scan-Geschwindigkeit erhöht werden, da in diesem Fall die Integrationszeit des Detektors 30 konstant bleiben kann.

Ein weiterer Vorteil der Lichtmodulation ist die Reduktion der Datenmenge für einen vollen dreidimensionalen Tiefenscan. Bei der Aufnahme eines dreidimensionalen Datensatzes einer lateralen Größe von 512 x 640 Pixel und einer Scantiefe von 1 mm in einem Gewebe mit Brechungsindex n = 1,4 entstehen ca. 6 GByte an Daten. Mit der oben beschriebenen Modulation der Intensität des Lichts 14 wird die Datenmenge auf 750 MByte reduziert.

Darüber hinaus müssen die unmittelbar gewonnenen Daten zusätzlich verarbeitet werden, um das Bildergebnis darzustellen. Auch hierbei ist die reduzierte Datenmenge sehr vorteilhaft, da hierdurch die Verarbeitungszeit deutlich reduziert wird und somit das Bildergebnis schneller vorliegt.

Vorzugsweise werden die Dopplerfrequenz und/oder die Modulationsfrequenz so gewählt, dass eine Periode des sich ergebenden Schwebungssignals ein ganzzahliges Vielfaches der minimalen Framezeit des Detektors 30 beträgt, d.h. dass die maximale Abtastrate des Detektors 30 ein ganzzahliges Vielfaches der Frequenz des Schwebungssignals beträgt.

Wählt man eine Periodenlänge der Modulation des Lichts 14 als minimale Framezeit des Detektors 30, so steigt die Scan-Geschwindigkeit um den Faktor 4 gegenüber der Scan-Geschwindigkeit bei nicht-modulierten Licht 14. Wählt man dagegen eine minimale Framezeit von zwei Perioden der Modulation, so steigt die Scan-Geschwindigkeit um den Faktor 8.

Fig. 6 c) zeigt die Einhüllende Eu bzw. Em des in den Figuren 6 a) bzw. 6 b) gezeigten Interferenzsignals bzw. Schwebungssignals bei unmoduliertem bzw. moduliertem Licht 14. Jeder Punkt P' der Einhüllenden Eu bzw. Em korrespondiert hierbei mit einem Abtastzeitpunkt P des zugehörigen Interferenzsignals bzw. Schwebungssignals.

Aus der jeweiligen Einhüllenden Eu bzw. Em werden Informationen abgeleitet, aus denen zunächst ein, zwei- und schließlich dreidimensionale Bilder der Probe 1 zusammengesetzt werden. Wie Versuche gezeigt haben, treten durch die durchgeführte Intensitätsmodulation trotz der deutlich geringeren Anzahl von Messpunkten P bzw. P' keine relevanten Informationsverluste gegenüber einem konventionellen System ohne Intensitätsmodulation auf.

Insgesamt wird durch die beschriebene Modulation der Intensität des eingekoppelten Lichts 14 die maximal mögliche Geschwindigkeit des Tiefenscans vervielfacht, ohne dass signifikante Informationsverluste bei der Signalauswertung auftreten.

5. Modulation der Empfindlichkeit des Detektorsystems

Das oben beschriebene Prinzip der Modulation der Intensität des in das erste Interferometer 10 eingekoppelten Lichts 14 bzw. des vom ersten Interferometer ausgegebenen Lichts des dritten Teilstrahls 4 lässt sich analog auf die Empfindlichkeit des Detektorsystems, welches u.a. den Detektor 30 und das Detektorobjektiv 31 umfasst, übertragen, indem die Empfindlichkeit des Detektorsystems, insbesondere des Detektors 30, für das zu erfassende Licht mit einer Frequenz moduliert wird, die vorzugsweise um einen bestimmten Betrag, insbesondere um bis zu 40 %, größer oder kleiner ist als die Dopplerfrequenz $f_D$.

Das von der Probe 1 reflektierte und auf den Detektor 30 treffende Licht überlagert sich hierbei mit der modulierten Empfindlichkeit des Detektorsystems 30, 31, so dass der Detektor 30 bei der Erfassung des auf den Detektor 30 treffenden Interferenzmusters anstelle eines hochfrequenten Interferenzsignals mit einer Vielzahl von Perioden ein niederfrequentes Schwebungssignal erzeugt, welches deutlich weniger Perioden aufweist als das hochfrequente Interferenzsignal. Bei der Abtastung dieser Schwebung sind daher deutlich weniger Abtastzeitpunkte pro Zeiteinheit erforderlich als bei einer Abtastung des hochfrequenten Interferenzsignals ohne die Modulation der Empfindlichkeit des Detektorsystems 30, 31.

Die Empfindlichkeit des Detektors 30 lässt sich z.B. direkt oder mit einem vor dem Detektor 30 angeordneten steuerbaren elektronischen Shutter modulieren. Alternativ oder zusätzlich können Eigenschaften eines optischen Elements im Detektorsystem, wie z.B. die Durchlässigkeit des Detektorobjektivs 31 für das von der Probe 1 reflektierte Licht, moduliert werden.

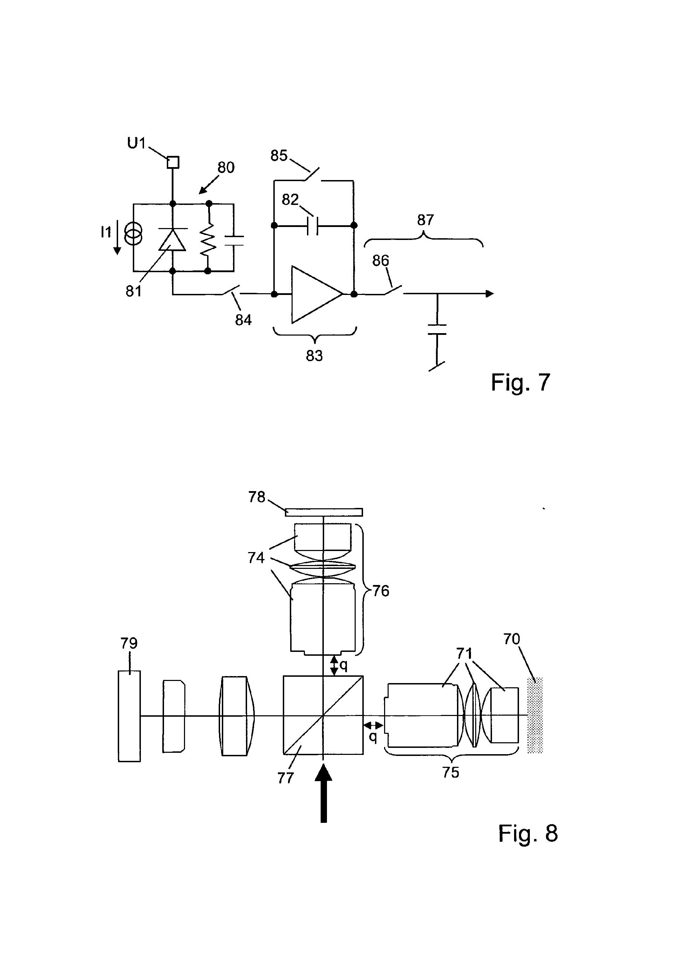

Die Funktionsweise der direkten Modulation der Empfindlichkeit des Detektors 30 wird anhand von Fig. 7, welche eine stark schematisierte elektrische Schaltung zeigt, näher erläutert. Jedes der Detektorelemente 80 eines CMOS-Detektors kann im Ersatzschaltbild vereinfacht als Photodiode 81 dargestellt werden, welche mit einer Spannung U1 vorgespannt ist. Optional sind ein ohmscher Widerstand und ein Kondensator zur Photodiode 81 parallelgeschaltet. Durch Bestrahlung des Detektorelements 80 mit Licht werden in der Photodiode 81 Ladungsträger erzeugt, die einen Stromfluss I1 auslösen, der in einem Kondensator 82 eines elektronischen Integrators 83 aufsummiert wird. Durch periodisches Ein- und Ausschalten dieser Integration mittels eines Schalters 84, welcher mit der Modulationsfrequenz $f_M$ gesteuert wird, wird die Ladungsmenge und damit die jeweils aktuell erfasste Lichtintensität mit der Modulationsfrequenz $f_M$ moduliert. Über eine Sample-and-Hold-Stufe 87 wird das entsprechende Detektorsignal abgegriffen und einer weiteren Verarbeitung zugeführt. Die weiteren Schalter 85 und 86 dienen zur Steuerung des Zurücksetzen der Integration bzw. des Abgreifens des Detektorsignals.

Analog zu der oben beschriebenen Modulation der Intensität des eingekoppelten bzw. ausgegebenen Lichts 14 bzw. 4 wird auch bei dieser Variante anstelle eines hochfrequenten Interferenzsignals ein niederfrequentes Schwebungssignal (vgl. Fig. 6 a) bzw. b)) erhalten, welches mit deutlich weniger Abtastzeitpunkten P abgetastet werden kann, ohne hierbei relevante Information zu verlieren. Bei einer gegebenen maximalen Abtastrate des Detektors 30 hat dies zur Folge, dass die maximale Geschwindigkeit für einen Tiefenscan des Systems um ein Vielfaches erhöht werden kann.

Wie bei der Modulation des eingekoppelten bzw. ausgegebenen Lichts 14 bzw. 4 (siehe Abschnitt 4), so wird auch hier durch eine geeignete Wahl der Frequenz der Modulation der Empfindlichkeit des Detektorsystems 30, 31 im Vergleich zu Systemen mit einer konstanten Detektorempfindlichkeit die Scan-Geschwindigkeit um den Faktor 4 oder sogar 8 erhöht.

Die Geschwindigkeit der Bewegung des zweiten Referenzspiegels 12 steht in einer festen Beziehung zur Frequenz der Modulation der Empfindlichkeit des Detektors 30 und ist vorzugsweise so gewählt, dass in eine Periodendauer des entstehenden Schwebungssignals eine ganzzahlige Anzahl von Abtastzeitpunkten, vorzugsweise vier Abtastzeitpunkte, passen (vgl. Fig. 6 b)).

Die auf diese Weise abgetasteten Schwebungssignale müssen vor einer Visualisierung noch verarbeitet werden, da in diesen Signalen noch die Interferenzinformation enthalten ist. Die wesentliche Information, die visualisiert werden soll, ist die Amplitude und Tiefenposition der jeweiligen Interferenz, nicht jedoch die Interferenzstruktur selbst. Dazu muss das Schwebungssignal demoduliert werden, d.h. es wird die Einhüllende des Schwebungssignals bestimmt (vgl. Em in Fig. 6 c)).

Da die Phase des Schwebungssignals im Allgemeinen unbekannt ist und diese sich auch für unterschiedliche Schwebungssignale aus unterschiedlichen Tiefen unterscheiden kann, wird ein digitaler Demodulationsalgorithmus eingesetzt, der unabhängig von der Phase ist. Vorzugsweise werden für die Abtastung des Interferenzsignals mit vier Abtastzeitpunkten pro Periode sog. 90° Phase Shift-Algorithmen verwendet. Hierdurch wird eine schnelle Demodulation des Schwebungssignals erreicht.

6. Messkopf mit asymmetrischem Linnik-Interferometer

Nachfolgend wird der Aufbau des Messkopfes, der das zweite Interferometer 20 umfasst, anhand der Figuren 4, 8 und 9 näher erläutert.

Bei dem zweiten Interferometer 20 handelt es sich um ein sog. Linnik-Interferometer. Fig. 8 zeigt ein Beispiel für einen typischen Aufbau eines solchen Linnik-Interferometers mit Strahlteiler 77, Referenzspiegel 78, Detektor 79 und Probe 70. Bei einem solchen Linnik-Interferometer sind einer Miniaturisierung grundsätzlich Grenzen gesetzt, was insbesondere für die Durchmesser der verwendeten optischen Elemente, wie z.B. der Objektive 75 und 76 bzw. der Linsen 71 und 74, und den geometrischen Aufbau gilt. Der Aufbau des Proben- bzw. Referenzobjektivs 75 bzw. 76 sowie deren Abstand q zum Strahlteiler 77 sind im Wesentlichen gleich.

Bei dem im vorliegenden OCT-System verwendeten Linnik-Interferometer sind die Abstände von Proben- und Referenzobjektiv 41 bzw. 46 zum zweiten Strahlteiler 24 (siehe Fig. 4) wegen der Fokusnachführung im Allgemeinen nicht für alle Scantiefen gleich. Dadurch kann es zu großen relativen optischen Pfadlängendifferenzen (OPD) zwischen Bildmitte und Bildrand des Proben- und Referenzbildes kommen. Diese können zur Folge haben, dass die Ortsfrequenz der zu erfassenden Interferenzstruktur größer wird als die Auflösung des zweidimensionalen Detektors 30, wodurch die Interferenz nicht mehr oder nur noch ungenügend zuverlässig nachgewiesen werden kann.

Zur Vermeidung dieser Nachteile werden im zweiten Interferometer 20 des vorliegenden OCT-Systems das Proben- und Referenzobjektiv 41 bzw. 46 unterschiedlich ("asymmetrisch") ausgeführt und aufeinander abgestimmt, wie nachfolgend anhand von Fig. 4 näher erläutert wird.

Der Abstand p des Probenobjektivs 41, insbesondere der Linsen 42, zum zweiten Strahlteiler 24 wird sehr klein gewählt. Für die obere Scan-Position, bei welcher das von einem in der Nähe der Oberfläche der Probe 1 liegenden Schnitt (vgl. Fig. 2 a) reflektierte Licht erfasst wird, beträgt der Abstand p vorzugsweise zwischen 1 und 3 mm. Dadurch können die Durchmesser der Linsen 42 bzw. 49 im Proben- und Referenzarm 22 bzw. 23 bei gleichzeitig hoher Lichtausbeute sehr klein gewählt werden.

Eine weitere Gruppe von Linsen 47 im Ausgangsarm 27 bildet zusammen mit dem Proben- bzw. Referenzobjektiv 41 bzw. 46 die Proben- bzw. Referenzoptik. Proben- und Referenzoptik sind telezentrisch auf der Seite der Probe 1 bzw. des dritten Referenzspiegels 25. Telezentrische Optiken zeichnen sich dadurch aus, dass der Objektabstand variiert werden kann und die Bildgröße trotzdem konstant bleibt. Dies wird durch eine Aperturblende erreicht.

Die numerische Apertur für die Abbildung der Probe 1 ist relativ groß, vorzugsweise etwa 0,3. Die numerische Apertur der Beleuchtung der Probe 1 dagegen ist kleiner als die numerische Apertur für die Abbildung der Probe 1 und hat vorzugsweise einen Wert von 0,2. Hierdurch wird zusammen mit dem telezentrischen Design der Proben- bzw. Referenzoptik der Vorteil erzielt, dass auch das an schräg stehenden Probenstrukturen reflektierte Licht noch vom Probenobjektiv 41 aufgesammelt wird, da der Akzeptanzwinkel des Probenobjektivs 41 größer ist als der Divergenzwinkel des Beleuchtungskegels. Wäre die numerische Apertur für Beleuchtung und Abbildung dagegen gleich groß, so würde bei der Reflexion an schräg stehenden Probenstrukturen weniger Licht aufgesammelt als bei der Reflexion an Strukturen, die senkrecht zur optischen Achse stehen.

Im Probenarm 22 wird die kleinere numerische Apertur für die Beleuchtung durch die Wahl des Beleuchtungsobjektivs 48 im Beleuchtungsarm 21 realisiert. Die numerische Apertur im Referenzarm 23 ist gleich oder etwas größer als die numerische Apertur des Beleuchtungsarms 21. Dies ist insbesondere bei dem hier verwendeten gefalteten Linnik-Interferometer von Vorteil, da hierdurch das Referenzobjektiv 46 relativ einfach an das Probenobjektiv 41 angepasst und darüber hinaus kompakt realisiert werden kann.

Der optische Weg durch die Linsen 49 des Referenzobjektivs 46 (einschließlich etwaiger Luftabstände zwischen den Linsen 49) ist kürzer als der optische Weg durch die Gruppe der Linsen 42 des Probenobjektivs 41.

Durch diese Maßnahmen wird erreicht, dass die Bildfeldwölbungen von Proben- und Referenzarm 22 bzw. 23 in der Mitte der genutzten Scantiefe weitestgehend identisch sind. Außerdem wird gewährleistet, dass die maximale optische Pfadlängendifferenz (OPD) zwischen Bildmitte und Bildrand am oberen bzw. unteren Ende des Tiefenscans klein genug ist, um eine Ortsfrequenz der Interferenzstruktur zu garantieren, die klein genug ist, um die Nyquist-Bedingung im Hinblick auf den Detektor 30 zu erfüllen. Dadurch ist die Ortsfrequenz der Interferenzstrukturen aus unterschiedlichen Tiefen im betrachteten Raumelement 33 der Probe 1 stets kleiner als die Auflösung des zweidimensionalen Detektors 30. Die Interferenzstrukturen werden dadurch in jeder Tiefe des betrachteten Raumelements 33 der Probe 1 stets mit hoher Zuverlässigkeit erfasst.

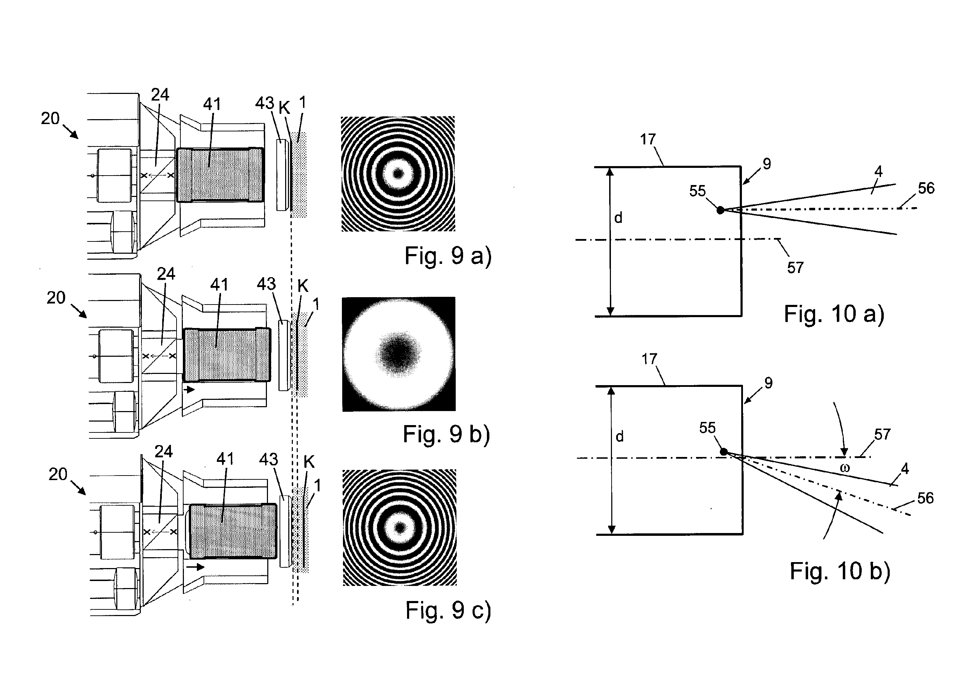

Dies ist in den Fig. 9 a) bis c) veranschaulicht, in welcher ein probenseitiger Ausschnitt des Querschnitts des zweiten Interferometers 20 zu drei unterschiedlichen Zeitpunkten während eines Tiefenscans dargestellt ist.

In einem ersten Zeitpunkt (siehe Fig. 9 a)) liegt das Kohärenz-Gate K in einer oberen Schicht 34 des betrachteten Raumelementes 33 der Probe 1 (vgl. Fig. 2 a)). Das Probenobjektiv 41 hat hierbei einen kleinen Abstand zum zweiten Strahlteiler 24 und einen relativ großen Abstand zur Materialschicht 43 bzw. zur Probe 1. Die hierbei erhaltene Interferenzstruktur ist im rechten Teil der Fig. 9 a) dargestellt und weist eine Periodenlänge auf, die dem Abstand zwischen jeweils zwei aufeinander folgenden hellen oder dunklen Ringen entspricht. Diese Periodenlänge ist größer als der Mitte-Mitte-Abstand (Pitch) der einzelnen Detektorelemente (Pixel) des Detektors 30, d.h. die Ortsfrequenz der Interferenzstruktur, welche der reziproken Periodenlänge entspricht, ist kleiner als die Auflösung des Detektors 30, welche dem reziproken Mitte-Mitte-Abstand der Pixel des Detektors 30 entspricht, wodurch die sog. Nyquist-Bedingung erfüllt ist. Hierdurch wird gewährleistet, dass die Interferenzstruktur zuverlässig vom Detektor 30 erfasst werden kann.

In einem zweiten Zeitpunkt (siehe Fig. 9 b)) liegt das Kohärenz-Gate K in einer mittleren Schicht 35 des betrachteten Raumelementes 33 der Probe 1 (vgl. Fig. 2 a)). Das Probenobjektiv 41 ist in einer Position, die etwas weiter vom zweiten Stahlteiler 24 entfernt und etwas näher an der Materialschicht 43 liegt als in Fig. 9 a). Die Interferenzstruktur weist in diesem Fall eine größere Periodenlänge auf als in Fig. 9 a), so dass auch zu diesem Zeitpunkt die Nyquist-Bedingung erfüllt ist.

In einem dritten Zeitpunkt (siehe Fig. 9 c)) liegt das Kohärenz-Gate K in der tiefsten Schicht 36 des betrachteten Raumelementes 33 der Probe 1 (vgl. Fig. 2 a)). Das Probenobjektiv 41 ist in einer Position, die noch weiter vom zweiten Stahlteiler 24 entfernt und noch näher an der Materialschicht 43 liegt als in Fig. 9 b). Die Interferenzstruktur weist in diesem Fall etwa dieselbe Periodenlänge wie zu dem in Fig. 9 a) dargestellten Zeitpunkt, so dass auch in dieser Tiefenscan-Position die Nyquist-Bedingung erfüllt ist.

Aufgrund der beschriebenen asymmetrischen Ausgestaltung des Proben- und Referenzobjektivs 41 bzw. 46 können unterschiedliche Abstände bzw. optische Wege p bzw. r des Proben- und Referenzobjektivs 41 bzw. 46 zum zweiten Strahlteiler 24 realisiert werden. Im dargestellten Beispiel kann dadurch das Probenobjektiv 41 im Abstand p nahe an den zweiten Strahlteiler 24 gebracht werden, wodurch sich kleine Durchmesser der Linsen 42 bei hoher Lichtausbeute realisieren lassen. Gleichzeitig kann das Referenzobjektiv 46 in einem deutlich größeren Abstand r (r > p) vom zweiten Strahlteiler 24 entfernt angeordnet werden, wodurch eine Faltung des zweiten Interferometers 20 ermöglicht wird, bei welcher Referenz- und Beleuchtungsarm 23 bzw. 21 jeweils um 90°gegenüber ihrer Lage in einem nicht gefalteten Linnik-Interferometer (vgl. Fig. 8) gekippt sind und dadurch parallel zum Probenarm 22 verlaufen.

Auf diese Weise wird eine sehr schlanke Form des Messkopfes erreicht und gleichzeitig gewährleistet, dass das Bild auf dem Detektor 30, das durch die Referenz- bzw. Probenoptik erzeugt wird, für alle Scantiefen gleich groß und gut überlagert ist.

Durch die oben beschriebene Ausführung des Referenzobjektivs 46 wird ein Teil des optischen Weges, der zur Faltung notwendig ist, kompensiert. Das Referenzobjektiv 46 ist also optisch kürzer als das Probenobjektiv 41. Dadurch wird die Ausführung des ersten Interferometers 10 einfacher, da sich hierdurch die beiden Interferometerarme des ersten Interferometers 10 nicht so stark voneinander unterscheiden müssen, um die Kohärenzbedingung für das Auftreten von Interferenz zu erfüllen.

Die Differenz der optischen Weglängen im Referenz- bzw. Probenarm 23 bzw. 22 ist vorzugsweise mindestens doppelt so groß wie die maximale Scantiefe Tm (siehe Fig. 3 a) und b)). Die maximale optische Scantiefe Tm gibt an, bis zu welcher Tiefe unterhalb der Oberfläche der Probe 1 die Kohärenzbedingung für das Auftreten einer Interferenz erfüllt und entsprechende Interferenzmuster erhalten werden. Dadurch wird eine eindeutige und einfache Zuordnung der Position des Referenzspiegels 12 im ersten Interferometer 10 zu einer bestimmten Tiefe in der Probe 1 gewährleistet.

7. Singlemode-Prämodulation und Multimode-Faser

Bei der hier bevorzugten Ausbildung des ersten Interferometers 10 in der sog. Freistahloptik wäre bei einer Verwendung der üblicherweise eingesetzten räumlich kurz- oder inkohärenten Lichtquellen ein relativ aufwändiges Objektiv im Bereich des Ausgangs 8 des ersten Interferometers 10 erforderlich, um das ausgehende Licht möglichst effizient in den ersten Lichtleiter 17 einzukoppeln und dabei Lichtverluste zu vermeiden. Dadurch würde nicht nur der optische Aufbau des zweiten Interferometers 20, welches für endoskopische Anwendungen möglichst kompakt zu gestalten ist, sondern auch der Aufbau der Optik des ersten Interferometers 10 beschränkt werden. Darüber hinaus ist eine gegebenenfalls erforderliche Steigerung der Lichtleistung bei den üblicherweise eingesetzten räumlich kurz- oder inkohärenten Lichtquellen begrenzt.

Um diese Nachteile zu vermeiden, wird im vorliegenden OCT-System als Lichtquelle 15 eine oder mehrere Singlemode-Lichtquellen mit jeweils hoher räumlicher Kohärenz eingesetzt, wie z.B. Superluminzeszenzdioden (SLEDs), Kurzpulslaser oder Superkontinuum-Laser. Das Licht 14 der Lichtquelle 15 wird in das erste Interferometer 10 eingekoppelt, wobei nur die sog. Gauss-Mode, die einer einzelnen Mode (Singlemode) entspricht, übertragen wird. Erst nach Durchlaufen des ersten Interferometers 10 wird die räumliche Kohärenz des eingekoppelten Lichts 14 zerstört, indem das Licht am Ausgang 8 des ersten Interferometers 10 in den ersten Lichtleiter 17, welcher eine sehr lange Multimode-Faser aufweist, eingekoppelt wird.

Bei einer Multimode-Faser handelt es sich um eine Faser, deren numerische Apertur und Kerndurchmesser es zulassen, dass sich bei einer bestimmten Wellenlänge des Lichts nicht nur eine Fasermode ausbilden kann, sondern viele verschiedene Fasermoden angeregt werden können. Ob eine Faser eine Singlemode-Faser oder eine Multimode-Faser ist, kann über die sog. V-Zahl V abgeschätzt werden:

$$V = \frac{\pi}{\lambda} \cdot d \cdot NA \,,$$

wobei $\lambda$ die Wellenlänge des in die Faser eingekoppelten Lichts, d der Kerndurchmesser der Faser und $NA$ die numerische Apertur der Faser angibt. Die Wellenlängen $\lambda$ des in die Faser eingekoppelten Lichts ist hierbei vorzugsweise identisch mit der mittleren Wellenlänge $\lambda_0$ des in das erste Interferometer 10 eingekoppelten Lichts 14. Ist die V-Zahl größer als etwa 2,4 so handelt es sich um eine Multimode-Faser.

Die im ersten Lichtleiter 17 vorzugsweise eingesetzte Multimode-Faser weist typische Längen in der Größenordnung von etwa 100 m auf und ist vorzugsweise zum überwiegenden Teil auf einer Wicklung 19 aufgewickelt, wie in Fig. 1 angedeutet. Der Kerndurchmesser der Multimode-Faser liegt vorzugsweise zwischen etwa 200 $\mu$m und etwa 400 $\mu$m.

Die sehr lange, dünne und vorzugsweise aufgewickelte Multimode-Faser kann im ersten Lichtleiter 17 optional mit einer relativ kurzen, dicken Faser (nicht dargestellt) kombiniert werden, deren Durchmesser im Bereich von etwa einem Millimeter und deren Länge im Bereich von Metern liegt.

Durch die Zerstörung der räumlichen Kohärenz des Lichts der Singlemode-Lichtquelle 15 wird vermieden, dass das von zwei unterschiedlichen Stellen in der Probe 1 reflektierte Licht interferieren kann, was auch als sog. kohärenter Cross-Talk bezeichnet wird.

Eine effiziente Unterdrückung des kohärenten Cross-Talk führt darüber hinaus zu einer effektiven Unterdrückung von unerwünschtem Streulicht, was im Falle einer Lichtquelle mit hoher räumlicher Kohärenz ebenfalls zur Interferenz beitragen würde und im Ergebnis zu einem unscharfen, verwaschenen Bild - ähnlich einem Bild hinter einer Milchglasscheibe - führen würde. Auf die oben beschriebene Weise erfolgt eine effiziente Zerstörung der räumlichen Kohärenz, wodurch die Detektion von Streulicht stark reduziert wird und schließlich ein scharfes Bild erhalten wird.

Die im ersten Interferometer 10 erzeugte Prä-Modulations-Information, d.h. die durch die Bewegung des zweiten Referenzspiegels 12 bewirkte spektrale Modulation des eingekoppelten Lichts 14, wird bei der Übertragung des Lichts durch die sehr lange Multimode-Faser des ersten Lichtleiters 17 jedoch nicht verändert. Dies wird dadurch gewährleistet, dass beide Arme des ersten Interferometers 10 in der Multimode-Faser identische Moden mit identischer Modenverteilung und identischen Phasen erzeugen.

Jede Mode für sich überträgt dann die Prä-Modulations-Information, wobei die einzelnen Moden nicht untereinander koppeln. Dies wird dadurch erreicht, dass die ersten und zweiten Teilstrahlen 2 bzw. 3 im ersten Interferometer 10 (siehe Fig. 1) kollinear und exakt zu einem dritten Teilstrahl 4 überlagert werden, bevor sie in die Multimode-Faser des ersten Lichtleiters 17 eintreten.

Der Eintritt des Lichts in die Multimode-Faser des ersten Lichtleiters 17 bestimmt hierbei die Anzahl und Verteilung der in der Multimode-Faser angeregten Moden. Zur besonders effizienten Zerstörung der räumlichen Kohärenz ist es vorteilhaft, eine Einkopplung zu wählen, bei der möglichst viele Moden angeregt werden. Dies kann insbesondere dadurch erfolgen, dass - wie in Fig. 10 a) und 10 b) dargestellt - der Fokus 55 der Lichtstrahlen, d.h. des dritten Teilstrahls 4, nicht auf der Facette 9, d.h. der Eintrittsebene, der Multimode-Faser des ersten Lichtleiters 17 liegt und/oder die Lichtstrahlen des dritten Teilstrahls 4 schräg in die Multimode-Faser des ersten Lichtleiters 17 eingekoppelt werden, wobei die optische Achse 56 der Lichtstrahlen gegen die Mittelachse 57 der Multimode-Faser des ersten Lichtleiters 17 verkippt ist und mit dieser einen Winkel ω einschließt, welcher vorzugsweise zwischen 5° und 40° liegt. Auf diese Weise wird einerseits die räumliche Kohärenz maximal unterdrückt und andererseits die Ausleuchtung der Facette 9 der Multimode-Faser homogener.

In den Figuren 10 a) und 10 b) ist darüber hinaus der Kerndurchmesser d der im ersten Lichtleiters 17 verwendeten Multimode-Faser eingezeichnet.

Die beschriebene Kombination der Einkopplung von hochkohärentem Licht 14 in das erste Interferometer 10 in Kombination mit der Einkopplung des anschließend im ersten Interferometer 10 spektral modulierten Lichts des dritten Teilstrahls 4 in den ersten Lichtleiter 17 ermöglicht es, die Optik im Bereich des Ausgangs 8 des ersten Interferometers 10 sehr einfach zu gestalten.

Da bei diesem Prinzip lichtstarke kohärente Lichtquellen, wie z.B. SLEDs, Kurzpulslaser oder Superkontinuum-Laser, als Lichtquelle 15 eingesetzt werden können, ist es möglich, deutlich höhere Leistungsdichten als mit den üblicherweise eingesetzten räumlich inkohärenten Lichtquellen zu erzielen. Das Signal-Rausch-Verhältnis der gewonnenen Bildinformation wird dadurch deutlich verbessert. Alternativ zu dem hier dargestellten und beschriebenen Freistrahl-Interferometer kann unter Einsatz dieses Prinzips das erste Interferometer 10 auch vollständig als Faser-Interferometer ausgelegt werden. Der Tiefenscan könnte dann z.B. statt über die Bewegung des zweiten Referenzspiegels 12 durch Dehnen einer Faser in einem der beiden Arme des ersten Interferometers 10 mittels eines sog. Fiber Stretcher durchgeführt werden.

8. Bildtransfer durch Faserbündel

Wie bereits näher erläutert, wird bei dem vorliegenden OCT-System ein Tiefenscan durch eine makroskopische Bewegung des Referenzspiegels 12 im ersten Interferometer 10 durchgeführt, während das von der Probe 1 reflektierte Licht über das zweite Interferometer 20 und den zweiten Lichtleiter 29 zum zweidimensionalen Detektor 30 weitergeleitet und von diesem erfasst wird.

Als zweiter Lichtleiter 29 wird ein aus einer Vielzahl von Einzelfasern zusammengesetztes Faserbündel eingesetzt. Faserbündel haben im Allgemeinen eine hohe numerische Apertur, welche technisch bedingt ist und im Bereich von 0,4 oder darüber liegt. Des Weiteren ist der Füllfaktor der Facette, d.h. des Eintritts- oder Austrittsquerschnitts, üblicher Faserbündel relativ gering. Beides würde bei der Übertragung des von der Probe 1 reflektierten Lichts vom zweiten Interferometer 20 zum Detektor 30 zu unerwünschten Lichtverlusten führen.

Um ein möglichst kompaktes OCT-System mit geringen Licht- und Informationsverlusten bei der Übertragung des von der Probe 1 reflektieren Lichts zu erhalten, wird das nachfolgend näher beschriebene Faserbündel eingesetzt.

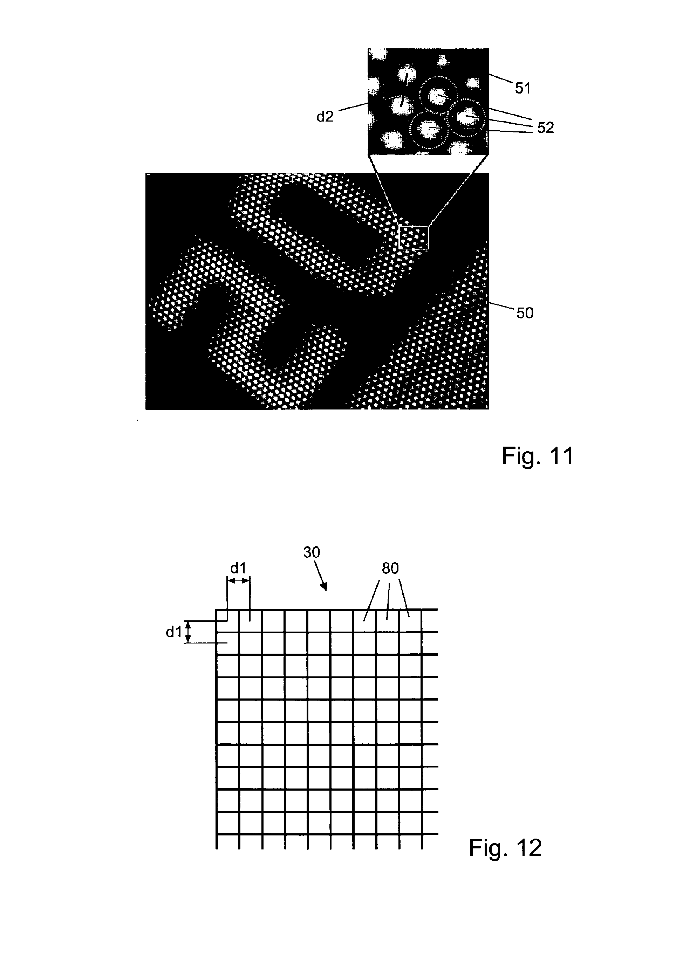

Fig. 11 zeigt einen Ausschnitt 50 aus der Facette des verwendeten Faserbündels, das sich - wie anhand des vergrößert dargestellten Teilbereichs 51 zu erkennen ist - aus einer Vielzahl von Einzelfasern 52 zusammensetzt, die einen Mitte-Mitte-Abstand d2 (sog. Faser-Pitch) aufweisen.

Fig. 12 zeigt einen Ausschnitt aus dem verwendeten Detektor 30, der eine Vielzahl von in einer Fläche angeordneten Detektorelementen 80 umfasst, die einen Mitte-Mitte-Abstand d1 (sog. Pixel-Pitch) aufweisen. Bei dem hier vorliegenden OCT-System ist der Faser-Pitch d2 der Einzelfasern 52 des Faserbündels kleiner als der Pixel-Pitch d1 der Detektorelemente 80 des Detektors 30.

Um ein möglichst großes Gesichtsfeld bei hoher räumlicher Auflösung zu ermöglichen, besteht das Faserbündel aus mindestens 100.000, vorzugsweise aus etwa 300.000, Einzelfasern 52. Die Anzahl der Detektorelemente 80 des Detektors 30 beträgt vorzugsweise etwa 328.000 und liegt damit in derselben Größenordnung wie die Anzahl der Einzelfasern 52.

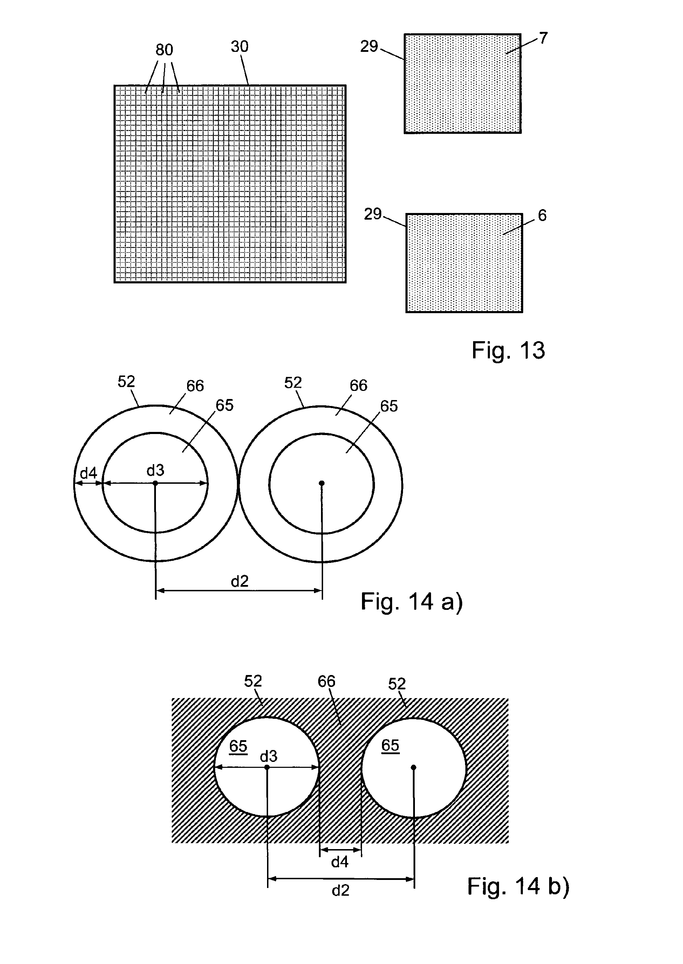

Wie in Fig. 13 dargestellt, ist die Form des Querschnitts des Faserbündels des zweiten Lichtleiters 29 im Bereich der Eintritts- und Austrittsfläche 7 bzw. 6 vorzugsweise an die Geometrie des Detektors 30 angepasst, wobei insbesondere die Form der Eintrittsfläche 7 auf der Seite des zweiten Interferometers 20 im Wesentlichen gleich der Form der Austrittsfläche 6 auf der Seite des Detektorobjektivs 31 bzw. Detektors 30 ist (siehe auch Fig. 1). Die jeweilige Form der Eintritts- und Austrittfläche 7 bzw. 6, insbesondere deren Seitenlängenverhältnis, ist hierbei im Wesentlichen mit der, vorzugsweise rechteckigen, Form des Detektors 30 identisch.

In Fig. 14 a) sind beispielhaft zwei Einzelfasern 52 des Faserbündels dargestellt. Die Einzelfasern 52 weisen einen Faserkern 65 und einen Fasermantel 66 auf. Bei den bevorzugt verwendeten Einzelfasern 52 des Faserbündels ist das Verhältnis d3/d4 der Dicken d3 bzw. d4 des jeweiligen Faserkerns 65 zum Fasermantel 66 (das sog. Core/Cladding-Verhältnis) so gewählt, dass sich ein möglichst hoher Füllfaktor bei möglichst geringen Lichtverlusten aufgrund von seitlich aus der Faser 52 austretendem Licht (sog. evaneszente Wellen) ergibt. Der Füllfaktor ist hierbei durch das Verhältnis der gesamten Querschnittfläche der Einzelfaser 52 zur Fläche des Faserkerns 65 gegeben.

Bei einer Wellenlänge des Lichts 14 von beispielsweise 1300 nm weist das verwendete Faserbündel vorzugsweise einen Faser-Pitch d2 von 11 $\mu$m, eine Mantelstärke d4 der Einzelfasern 52 von 1,7 $\mu$m und einem Kerndurchmesser d3 von 6,8 $\mu$m auf. Der Durchmesser der Einzelfaser 52, der sich aus der Summe des Kerndurchmessers d3 und der doppelten Mantelstärke d4 ergibt, beträgt in diesem Fall 10,2 $\mu$m und ist damit etwas geringer als der Faser-Pitch d2, da beim Herstellungsprozess des Faserbündels noch ein zweiter Mantel (nicht dargestellt) um jede Einzelfaser 52 erzeugt wird.

In Fig. 14 b) ist eine Variante der in Fig. 14 a) gezeigten Ausgestaltung der Einzelfasern 52 dargestellt. In dieser Variante sind die einzelnen Faserkerne 65 der Einzelfasern 52 in eine Matrix 66 aus Glas oder Kunststoff eingebettet, die jeweils den Fasermantel jedes einzelnen Faserkerns 65 bildet. Bei dieser Variante haben jeweils zwei benachbarte Einzelfasern 52 einen Teil ihres Fasermantels gemeinsam. Der Abstand d4 benachbarter Faserkerne 64, welcher der Manteldicke entspricht, kann hierdurch gegenüber den oben beschriebenen Einzelfasern mit einem jeweils eigenen Fasermantel reduziert werden, wobei das Auftreten evaneszenter Wellen weiterhin effizient unterdrückt wird. Das Flächenverhältnis der Faserkernfläche zur gesamten Faserfläche wird hierdurch besonders groß. Der Quotient aus dem Kerndurchmesser d3 und der Manteldicke d4 liegt hierbei im Bereich zwischen etwa 5 und 8.

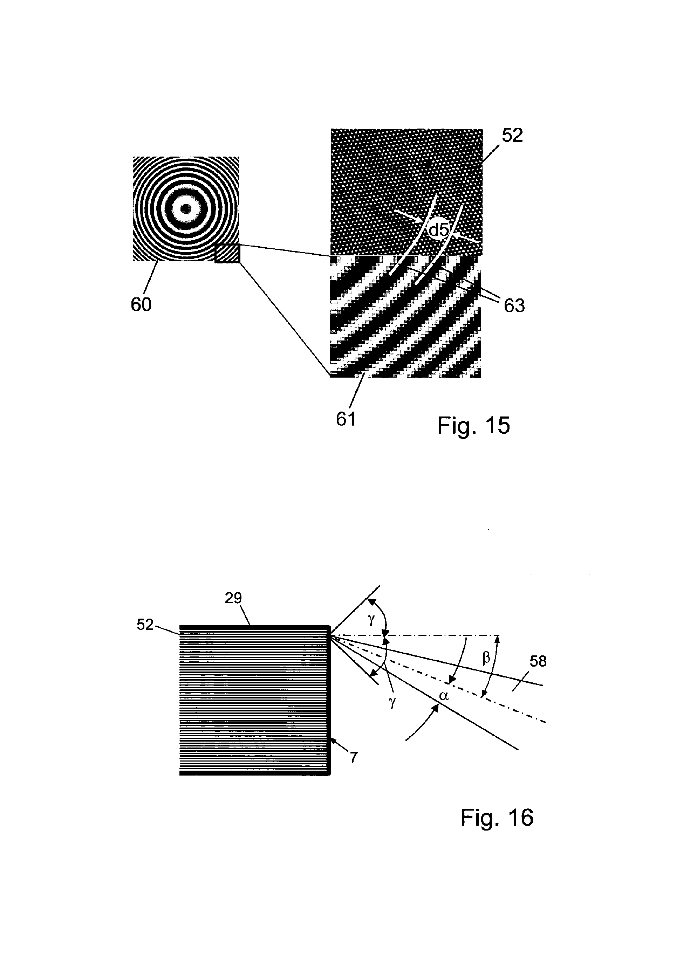

Das zweite Interferometer 20 ist so aufgebaut, dass sich für alle Scantiefen ein laterales Interferenzmuster ergibt, dessen Ortsfrequenz niedriger ist als die Ortsfrequenz der Einzelfasern 52 des Faserbündels, wobei insbesondere die Nyquist-Bedingung erfüllt sein muss. Dies ist in Fig. 15 veranschaulicht. Wie im vergrößerten Ausschnitt 61 des lateralen Interferenzmusters 60 zu erkennen ist, ist die Länge einer Periode zwischen zwei aufeinander folgenden Interferenzminima 63 (dunkle Ringe) des Interferenzmusters 60 um ein Vielfaches größer als der Mitte-Mitte-Abstand (Faser-Pitch) der Einzelfasern 52 des Faserbündels, dessen Eintrittsfläche 6 (siehe auch Fig. 1) hier ausschnittsweise und in entsprechender Vergrößerung argestellt ist. Entsprechend ist die Ortsfrequenz des Interferenzmusters 60 deutlich kleiner als die Ortsfrequenz der Einzelfasern 52 des Faserbündels.

Gegenüber aus dem Stand der Technik bekannten Systemen, bei denen der Detektor in das Interferometer eingebaut ist, werden durch die Verwendung des oben beschriebenen Faserbündels mehrere Vorteile erzielt, die nachfolgend näher beschrieben werden.

Der Pixel-Pitch d1 von InGaAs CMOS-Detektoren, die für Licht mit Wellenlängen im Bereich um etwa 1300 nm empfindlich sind, kann aus technischen Gründen nicht deutlich kleiner als 20 $\mu$m werden. Das im vorliegenden OCT-System bevorzugt verwendete Faserbündel weist einen Faser-Pitch d2 von 10 $\mu$m auf und weist daher bei gleicher Auflösung einen erheblich kleineren Querschnitt auf als der Detektor. Dies erlaubt einen deutlich kompakteren Aufbau des Messkopfes gegenüber Systemen, bei denen der Detektor in den Messkopf eingebaut ist.

Darüber hinaus wäre bei den genannten Systemen aus dem Stand der Technik aufgrund der geforderten, sehr hohen Abtastraten des Detektors eine Übertragung von Daten mit extrem hoher Geschwindigkeit vom Messkopf zur nachgeschalteten Elektronik erforderlich. Außerdem müssten A/D-Wandler in den Messkopf integriert werden. Diese Nachteile entfallen bei der hier beschriebenen Weiterleitung der von der Probe 1 erhaltenen Bildinformation durch den als Faserbündel ausgestalteten zweiten Lichtleiter 29 zu einem vom zweiten Interferometer 20 separaten Detektor 30.

Da bei dem vorliegenden OCT-System somit keine Elektronik zur Bilderfassung und/oder -verarbeitung im Messkopf erforderlich ist, gibt es keine Verlustwärme, die zu einer unerwünschten Erwärmung des Messkopfes führen könnten.

Da im zweiten Lichtleiter 29 vorzugsweise ein Faser-Pitch d2 (z.B. 11 $\mu$m) gewählt wird, der kleiner ist als der kleinstmögliche Pixel-Pitch d1 (meist größer oder gleich 20 $\mu$m) des Detektors 30, kann eine Vergrößerung des von der Probe 1 erhaltenen Bildes im Messkopf bei gleicher lateraler Auflösung gegenüber Systemen aus dem Stand der Technik reduziert werden, was eine einfachere und kleinere Optik im zweiten Interferometer 20 ermöglicht.

Zur Erhöhung der Lichtausbeute bei der Licht- bzw. Bildinformationsübertragung von der Probe 1 bzw. vom dritten Referenzspiegel 25 zum Detektor 30 ist eine Anpassung der numerischen Aperturen einzelner Komponenten des vorliegenden OCT-Systems vorgesehen, insbesondere der Aperturen des Probenobjektivs 41 und der Linsen 47 im Ausgangsarm 27 sowie der Aperturen des Referenzobjektivs 46 und des Faserbündels des zweiten Lichtleiters 29, des Detektorobjektivs 31 sowie des Detektors 30. Dies wird nachfolgend anhand der Figuren 1, 4 und 16 näher erläutert.

Fig. 16 zeigt einen Abschnitt des aus einer Vielzahl von Einzelfasern 52 zusammengesetzten zweiten Lichtleiters 29 im Bereich der Eintrittsfläche 7. Ein aus dem zweiten Interferometer 20 austretendes, konvergentes Lichtbündel 58 weist einen Aperturwinkel $\alpha$ auf und trifft in einem Einfallswinkel $\beta$ gegen die Normale der Eintrittsfläche 7 auf den Lichtleiter 29. Die Einzelfasern 52 des zweiten Lichtleiters 27 weisen einen Aperturwinkel $\gamma$ auf, innerhalb dessen sie auftreffendes Licht erfassen können. Der Aperturwinkel $\gamma$ ist durch die numerische Apertur der Einzelfasern 52 gegeben.

Zur Gewährleistung einer möglichst hohen Lichtausbeute ist vorzugsweise vorgesehen, dass die Summe aus dem Aperturwinkel $\alpha$ des Lichtbündels 58 und dem Einfallswinkel $\beta$ kleiner ist als der Aperturwinkel $\gamma$ der Einzelfasern 52 des Faserbündels 29: $\alpha + \beta < \gamma$. Dadurch wird sichergestellt, dass das gesamte Licht des Lichtbündels 58, das auf eine Einzelfaser 52 trifft, in diese eintreten und bis zur Austrittsfläche 6 des zweiten Lichtleiters 29 transportiert wird.

Die hierfür erforderlichen Aperturwinkel $\alpha$ und Einfallswinkel $\beta$ des Lichtbündels 58 werden durch eine entsprechende Ausgestaltung des Proben- und/oder Referenz- und/oder Ausgangsobjektivs 41, 46 bzw. 47 realisiert. Dies wird insbesondere dadurch erreicht, dass die beiden Objektiv-Kombinationen aus Proben-und Ausgangsobjektiv 41/47 bzw. Referenz- und Ausgangsobjektiv 46/47 vergrößernd abbilden, d.h. der Aperturwinkel $\alpha$ des Lichtbündels 58 im Bereich der Eintrittsfläche 7 des Faserbündels ("Bildseite") ist kleiner als der Aperturwinkel (nicht dargestellt) auf der Seite der Probe 1 ("Objektseite"). Dadurch lässt sich auf einfache Weise ein großer Aperturwinkel auf der Seite der Probe 1 realisieren, wodurch eine hohe Lichtsammeleffizienz erreicht wird. Zusammen mit der verlustfreien Lichteinkopplung in das Faserbündel des zweiten Lichtleiters 29 wird hierdurch eine insgesamt sehr hohe Lichtausbeute bei der Erfassung des von der Probe 1 reflektierten Lichts gewährleistet und damit eine hohe Bildqualität erreicht.

Alternativ oder zusätzlich ist zur Steigerung der Lichtausbeute eine Anpassung faserbündelseitigen numerischen Apertur des Detektorobjektivs 31 auf die numerische Apertur des Faserbündels des zweiten Lichtleiters 29 vorgesehen. Der Aperturwinkel des Detektorobjektivs 31 ist hierbei größer als der Aperturwinkel $\gamma$ der Einzelfasern 52 des Faserbündels.

Vorzugsweise ist das Detektorobjektiv 31 auf der Seite des Faserbündels telezentrisch. Hierdurch kann auf einfache Weise der Abstrahlcharakteristik des Faserbündels Rechnung getragen werden. Der Feldwinkel auf der Ausgangsfläche 6 ist für jede Position auf der Ausgangsfläche 6 gleich Null.

Mit zunehmendem Einfallswinkel der Lichtstrahlen auf den Detektor 30 wird die vom Detektor 30 erfasste Lichtleistung geringer. Zur Gewährleistung einer möglichst hohen Lichtausbeute ist daher vorgesehen, den Einfallswinkel der Lichtstrahlen auf den Detektor 30 möglichst gering zu halten. Dies wird vorzugsweise durch eine vergrößernde Abbildung des Faserbündels des zweiten Lichtleiters 29 auf den Detektor 30 und ein telezentrisches Design des Detektorobjektivs 31 auf der Seite des Detektors 30 erreicht.

Ein weiterer Vorteil bei der Verwendung des beschriebenen Faserbündels zur Bildübertragung besteht darin, dass die Gesamtvergrößerung M des Systems in zwei Schritte aufgeteilt werden kann, nämlich in eine erste Vergrößerung M1 im Messkopf, d.h. im zweiten Interferometer 20, und eine zweite Vergrößerung M2 im Detektorobjektiv 31. Dadurch kann die erste Vergrößerung M1 der Objektive 41, 47 und 47 im Messkopf kleiner sein als die für die nominelle Auflösung des OCT-Systems notwendige Gesamtvergrößerung M. Das folgende Beispiel soll dies verdeutlichen: Bei einem Pixel-Pitch von 20 $\mu$m, einem Faser-Pitch von 10 s$\mu$m und einer nominellen Auflösung von 2,5 $\mu$m kann durch das wie oben beschrieben ausgestaltete Faserbündel des zweiten Lichtleiters 29 eine Vergrößerung M1 = 4 im Messkopf und eine Vergrößerung M2 = 2 im Detektorobjektiv 31 realisiert werden, um eine Gesamtvergrößerung M = M1 x M2 = 8 zu erhalten. Ohne eine Bildübertragung durch das beschriebene Faserbündel müsste dagegen im Messkopf eine Vergrößerung erzeugt werden, die gleich der Gesamtvergrößerung M = 8 wäre.

Die Verwendung des oben beschriebenen Faserbündels hat somit den Vorteil, dass die Gesamtvergrößerung M nicht allein durch die Objektive des zweiten Interferometers 20 bewerkstelligt werden muss, so dass das Proben- und/oder Referenz- und/oder Ausgangsobjektivs 41, 46 bzw. 47 des Messkopfes einfacher und platzsparender aufgebaut werden kann bzw. können, wodurch der Messkopf insgesamt wesentlich kompakter gestaltet werden kann.

Wie in dem in Fig. 4 gezeigten Beispiel eines zweiten Interferometers 20 kann hierdurch der mittlere Durchmesser D1 des Probenobjektivs 41 bzw. der Linsen 47 des Ausgangsobjektivs des zweiten Interferometers 20 vorzugsweise kleiner gewählt werden als der Durchmesser D2 des zweiten Lichtleiters 29 im Bereich der Eintrittsfläche 7: D1 < D2.

9. Betriebsmodi des OCT-Systems

Das oben beschriebene OCT-System kann in drei unterschiedlichen Betriebsmodi betrieben werden. Bei den Betriebsmodi handelt es sich um zwei Echtzeitmodi, in denen OCT-Bilder einer Probe mit einer hohen Rate von etwa 5 bis 10 Bildern pro Sekunde erzeugt werden, sowie einem statischen Betriebsmodus.

Im ersten Betriebsmodus, dem Echtzeitmodus 1, werden in Echtzeit zweidimensionale Tiefenschnitte der Probe 1 erzeugt (sog. Slices). Dies wird dadurch realisiert, dass als Detektor 30 eine CMOS-Kamera verwendet wird, welche die Einstellung eines sog. Window of Interest (WOI) zulässt, bei welcher lediglich eine Teilfläche des Detektors 30 für Licht sensitiv ist und dieses in entsprechende Detektorsignale umwandelt. Die Reduzierung der sensitiven Kamerafläche ist verbunden mit einer deutlichen Erhöhung der Kamerageschwindigkeit; es können bei dieser Einstellung mehr Kamerabilder pro Sekunde erzeugt werden als im Vollbild-Modus.

Im Echtzeitmodus 1 wird vorzugsweise ein WOI gewählt, das in einer Richtung der gesamten Kameralänge bzw. -breite entspricht (z.B. 640 Pixel) und in der anderen Richtung die - durch den Typ der jeweiligen Kamera gegebene - minimal mögliche Anzahl von Pixel aufweist (z.B. 4 Pixel). Dadurch wird die Geschwindigkeit der Kamera so weit erhöht, dass OCT-Bilder in Echtzeit aufgenommen werden können.

Dies wird vorzugsweise in Kombination mit der Modulation der Intensität des in das erste Interferometer 10 eingekoppelten bzw. vom ersten Interferometer 10 ausgegebenen Lichts 14 bzw. 4 oder der Modulation der Empfindlichkeit des Detektorsystems 30, 31 (siehe Abschnitt 3 bzw. 4 oben) erreicht.

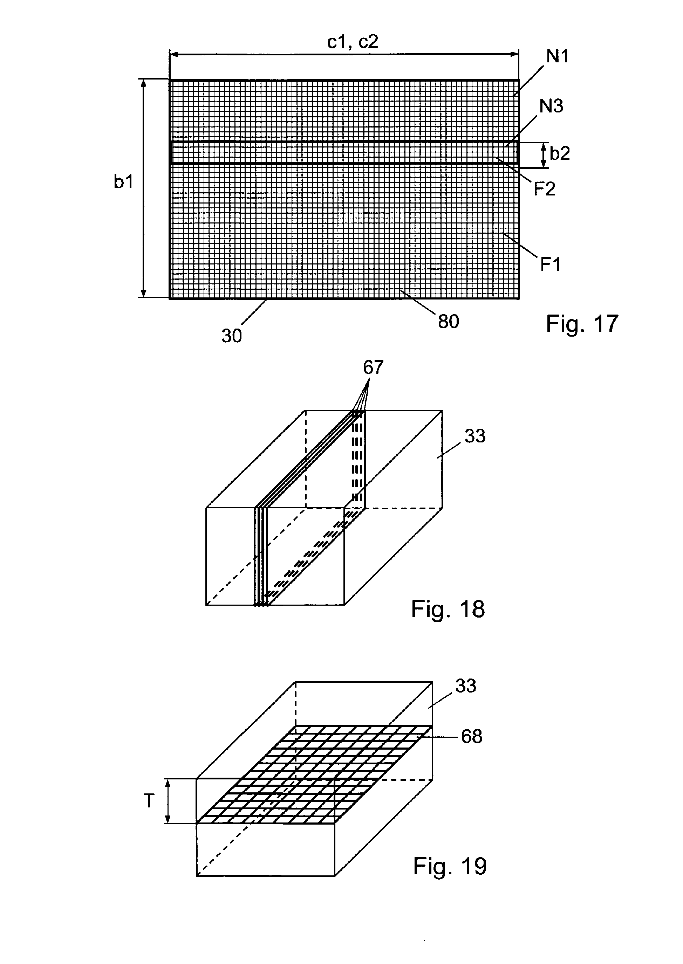

Fig. 17 zeigt eine Detektorfläche F1, welche sich aus einer ersten Anzahl N1 von Detektorelementen 80 zusammensetzt und eine Länge c1 und eine Breite b1 aufweist. Bei der o.g. Einstellung eines WOI wird Licht lediglich von den in einer Teilfläche F2 der Detektorfläche F1 befindlichen Detektorelementen 80 erfasst und in entsprechende Detektorsignale umgewandelt. Die zweite Anzahl N2 der Detektorelemente 80 der Teilfläche F2 ist kleiner als die erste Anzahl N1 der Detektorelemente 80 der gesamten Detektorfläche F1. Die Längen c1 und c2 der Detektorfläche F1 bzw. Teilfläche F2 sind gleich groß, während die Breiten b1 und b2 der Detektorfläche F1 bzw. Teilfläche F2 unterschiedlich sind.

Im gezeigten Beispiel ist die Teilfläche F2 nur vier Pixel breit, wohingegen die Detektorfläche F1 512 Pixel breit ist. Die sensitive Fläche der Detektorfläche F1 wird also um einen Faktor von 128 reduziert, was die für die Erfassung von Interferenzmustern und deren Umwandlung in entsprechende Detektorsignale erforderliche Zeitdauer erheblich verkürzt.

Wie in Fig. 18 dargestellt ist, werden in diesem Beispiel anstelle eines vollen dreidimensionalen Tomogramms nur vier (entsprechend den vier Pixelreihen der Teilfläche F2) zweidimensionale Tiefenschnitte 67 aus dem betrachteten Raumelement 33 der Probe 1 erhalten.

Im zweiten Betriebsmodus, dem Echtzeitmodus 2, werden - wie in Fig. 19 dargestellt - zweidimensionale Tomogramme 68 aus einer bestimmten Tiefe T des betrachteten Raumelements 33 der Probe 1 erzeugt, wobei die Tiefe T frei wählbar ist. Hierbei wird die gesamte Detektorfläche F1 des Detektors 30 für die Erfassung des von der Probe 1 reflektierten Lichts und dessen Umwandlung in entsprechende Detektorsignale genutzt, wobei jedoch nur jeweils maximal fünf Kamerabilder zur Berechnung eines Tomogramms 68 herangezogen werden. Dazu wird der erste Referenzspiegel 11 im ersten Interferometers 10 mit einer Amplitude von etwa 1 $\mu$m periodisch bewegt, während bis zu fünf Kamerabilder aufgenommen werden, die dann zu einem OCT-Bild verrechnet werden. Auf diese Weise können Tomogramme 68 mit hoher Wiederholungsrate erzeugt werden.

Durch eine makroskopische Bewegung des zweiten Referenzspiegels 12 ggf. in Kombination mit der Fokusnachführung (siehe Abschnitt 1 bzw. 2 oben) kann die Tiefe T, aus der das Tomogramm 68 gewonnen wird, frei gewählt werden.

Im dritten Betriebsmodus, dem statischen Modus, wird ein vollständiger dreidimensionaler Datensatz mit Hilfe der makroskopischen Bewegung des zweiten Referenzspiegels 12 in Kombination mit der Fokusnachführung aufgenommen. Einzelheiten hierzu sind insbesondere den Abschnitten 1 und 2 zu entnehmen. Durch die verschiedenen Betriebsmodi kann das OCT-System eine Reihe unterschiedlicher Anforderungen erfüllen. Die Funktionalitäten bei der Untersuchung von Proben, beispielsweise beim Auffinden relevanter Stellen in der Probe, wird dadurch erheblich erweitert.

10. Weitere erfinderische Aspekte des Systems bzw. Verfahrens zur OCT

Das vorstehend näher beschriebene System bzw. Verfahren zur OCT weist einzelne Merkmale oder Merkmalskombinationen auf, durch die das System bzw. Verfahren insbesondere im Aufbau einfacher und kompakter sowie in der Handhabung und Bilderfassung schneller und zuverlässiger gemacht wird, ohne dass hierbei alle im Oberbegriff und/oder Kennzeichenteil der unabhängigen Ansprüche aufgeführten Merkmale erforderlich sind. Diese Merkmale bzw. Merkmalskombinationen werden ebenfalls als Erfindung angesehen.

Als Erfindung wird insbesondere ein System zur optischen Kohärenztomographie mit

- mindestens einem Interferometer zur Ausgabe von Licht, mit dem eine Probe bestrahlt wird, und
- einem Detektor zur Erfassung von Licht, welches von der Probe reflektiert wird, angesehen, wobei das System durch ein oder mehrere Merkmale gekennzeichnet ist, die vorstehend, insbesondere in den Abschnitten 1 bis 9 und/oder im Zusammenhang mit den Figuren 1 bis 19, näher beschrieben wurden.

Das diesem System entsprechende Verfahren wird ebenfalls als Erfindung angesehen.

Die Bestrahlung der Probe mit dem vom Interferometer ausgegebenen Licht erfolgt entweder indirekt, d.h. über ein weiteres Interferometer, welches zwischen dem Interferometer und der Probe liegt, oder direkt, d.h. ohne ein zwischen dem Interferometer und der Probe befindliches weiteres Interferometer.

Die Erfassung des von der Probe reflektierten Lichts durch den Detektor erfolgt entweder indirekt, d.h. über ein weiteres Interferometer, welches zwischen der Probe und dem Detektor liegt, oder direkt, d.h. ohne ein zwischen dem Detektor und der Probe befindliches weiteres Interferometer.

**Patentansprüche**

1. System zur optischen Kohärenztomographie mit

   - einem Interferometer (10, 20) zur Ausgabe von Licht, mit dem eine Probe (1) bestrahlt wird, wobei das Interferometer (10, 20) einen Strahlteiler (13, 24) und mindestens einen Reflektor (11, 12, 25) umfasst, und
   - einem Detektor (30) mit einer ersten Anzahl (N1) von in einer ersten Fläche (F1) angeordneten Detektorelementen (80) zur Erfassung von Licht, welches von der Probe (1) reflektiert wird,

   **dadurch gekennzeichnet, dass**
   das System in einem ersten Modus, insbesondere einem ersten Echtzeitmodus, betrieben werden kann, in welchem von der Probe (1) reflektiertes Licht nur von einer zweiten Anzahl (N3) von Detektorelementen (80) des Detektors (30) erfasst und in entsprechende Detektorsignale umgewandelt wird, wobei die zweite Anzahl (N3) der Detektorelemente (80) kleiner ist als die erste Anzahl (N1) der Detektorelemente (80), und
   das System in einem zweiten Modus, insbesondere einem zweiten Echtzeitmodus, betrieben werden kann, in welchem während einer Veränderung des optischen Abstandes des Reflektors (11, 12, 25) zum Strahlteiler (13, 24) das von der Probe (1) reflektierte Licht von den Detektorelementen (80) des Detektors (30) mehrmals, insbesondere höchstens fünfmal, erfasst wird, wodurch ein zweidimensionaler Schnitt (68) durch ein Raumelement (33) der Probe (1) in einer vorgegebenen Tiefe (T) der Probe (1) erhalten wird.

2. System nach Anspruch 1, wobei die zweite Anzahl (N3) der Detektorelemente (80) höchstens ein Viertel der ersten Anzahl (N1) der Detektorelemente (80) beträgt: $N3 \leq \frac{1}{4} N1$.

3. System nach Anspruch 1 oder 2, wobei die zweite Anzahl (N3) der Detektorelemente (80) in einer zweiten Fläche (F2) angeordnet ist, welche eine zusammenhängende Teilfläche der ersten Fläche (F1) bildet.

4. System nach Anspruch 3, wobei die erste Fläche (F1) eine erste Breite (b1) und eine erste Länge (c1) und die zweite Fläche (F2) eine zweite Breite (b2) und eine zweite Länge (c2) aufweist, wobei die erste und die zweite Länge (c1 bzw. c2) im Wesentlichen identisch sind und die zweite Breite (b2) kleiner ist als die erste Breite (b1).

5. System nach einem der vorangehenden Ansprüche, wobei der optische Abstand (I) des Reflektors (11, 12, 25) zum Strahlteiler (13, 24) um einen optischen Weg (L) veränderbar ist, der wesentlich, insbesondere mindestens 100 mal, größer ist als die mittlere Wellenlänge ($\lambda_0$) von in das Interferometer (10, 20) eingekoppeltem Licht (14): $L \gg \lambda_0$, wobei der optische Abstand (I) des Reflektors (11, 12, 25) zum Strahlteiler (13, 24) um einen optischen Weg von insbesondere 0,1 mm bis zu mehreren Millimetern veränderbar ist.

6. System nach Anspruch 5, wobei während der Veränderung des optischen Abstands (I) des Reflektors (11, 12, 25) zum Strahlteiler (13, 24) um den optischen Weg (L) das von der Probe (1) reflektierte Licht nur von der zweiten Anzahl (N3) von Detektorelementen (80) des Detektors (30) mehrmals erfasst wird, wodurch mehrere zweidimensionale Tiefenschnitte (67) durch ein Raumelement (33) der Probe (1) erhalten werden.

7. System nach Anspruch 5 oder 6, wobei die Tiefe (T) des zweidimensionalen Schnitts (68) durch das Raumelement (33) der Probe (1) durch den optischen Abstand (I) des Reflektors (11, 12, 25) zum Strahlteiler (13, 24) vorgebbar ist.

8. System nach einem der vorangehenden Ansprüche, wobei der optische Abstand des Reflektors (11, 12, 25) zum Strahlteiler (13) um einen weiteren optischen Weg (L') veränderbar ist, welcher höchstens das Zehnfache der mittleren Wellenlänge ($\lambda_0$) des in das Interferometer (10) eingekoppelten Lichts (14) beträgt: $L' \leq 10 \cdot \lambda_0$.

9. System nach einem der vorangehenden Ansprüche, wobei das System in einem dritten Modus betrieben werden kann, in welchem während der Veränderung des optischen Abstandes (I) des Reflektors (11, 12, 25) zum Strahlteiler (13, 24) um den optischen Weg (L) das von der Probe (1) reflektierte Licht von den Detektorelementen (80) des Detektors (30) mehrmals erfasst wird, wodurch das von mehreren zweidimensionalen Schnitten (34, 35, 36) in unterschiedlichen Tiefen (T1, T2, T3) der Probe (1) reflektierte Licht erfasst wird.

10. System nach einem der vorangehenden Ansprüche mit einem Probenobjektiv (41), durch welches vom Interferometer (10, 20) ausgegebenes Licht in einem auf oder in der Probe (1) liegenden Fokus (F) fokussiert wird, wobei während der Veränderung des optischen Abstands (I) des Reflektors (11, 12, 25) zum Strahlteiler (13, 24) das in mehreren unterschiedlichen Tiefen (T1, T2, T3) der Probe (1) jeweils reflektierte Licht vom Detektor (30) erfasst wird und gleichzeitig die Abbildungseigenschaften des Probenobjektivs (41) in der Weise gesteuert werden, dass der Fokus (F) im Bereich (K) der jeweiligen Tiefe (T1, T2, T3) der Probe (1) liegt.

11. System nach einem der vorangehenden Ansprüche, wobei die Intensität von Licht (14 bzw. 4), welches in das Interferometer (10) eingekoppelt oder vom Interferometer (10) ausgegeben wird, mit einer Modulationsfrequenz ($f_M$) moduliert wird.

12. System nach einem der Ansprüche 1 bis 10, wobei ein Detektorsystem (30, 31) vorgesehen ist, welches den Detektor (30) umfasst, wobei die Empfindlichkeit des Detektorsystems (30, 31) für das von der Probe (1) reflektierte und auf den Detektor (30) treffende Licht mit einer Modulationsfrequenz ($f_M$) moduliert wird.

13. System nach Anspruch 11 oder 12, wobei die Modulationsfrequenz ($f_M$) ungleich der Dopplerfrequenz ($f_D$) ist, wobei die Dopplerfrequenz ($f_D$) durch das Zweifache des Verhältnisses der Geschwindigkeit (v) der Veränderung des optischen Abstands des Reflektors (11, 12, 25) zum Strahlteiler (13, 24) um den optischen Weg (L) bzw. den weiteren optischen Weg (L') zur mittleren Wellenlänge ($\lambda_0$) des in das Interferometer (10) eingekoppelten Lichts (14) gegeben ist: $f_M \neq f_D = 2 \cdot v / \lambda_0$.

14. System nach einem der vorangehenden Ansprüche, wobei im ersten Modus, insbesondere im ersten Echtzeitmodus, und/oder im zweiten Modus, insbesondere im zweiten Echtzeitmodus, Bilder der Probe (1) mit einer Rate von etwa 5 bis 10 Bildern pro Sekunde erzeugt werden können.

15. Verfahren zur optischen Kohärenztomographie, bei welchem

    - von einem Interferometer (10, 20) Licht ausgegeben wird, mit dem eine Probe (1) bestrahlt wird, wobei das Interferometer (10, 20) einen Strahlteiler (13) und mindestens einen Reflektor (11, 12, 25) umfasst, und

- von der Probe (1) reflektiertes Licht von einem Detektor (30) erfasst wird, welcher eine erste Anzahl (N1) von in einer ersten Fläche (F1) angeordneten Detektorelementen (80) umfasst,

**dadurch gekennzeichnet, dass**

in einem ersten Modus, insbesondere einem ersten Echtzeitmodus, von der Probe (1) reflektiertes Licht nur von einer zweiten Anzahl (N3) von Detektorelementen (80) des Detektors (30) erfasst und in entsprechende Detektorsignale umgewandelt wird, wobei die zweite Anzahl (N3) der Detektorelemente (80) kleiner ist als die erste Anzahl (N1) der Detektorelemente (80), und in einem zweiten Modus, insbesondere einem zweiten Echtzeitmodus, während einer Veränderung des optischen Abstandes des Reflektors (11, 12, 25) zum Strahlteiler (13, 24) das von der Probe (1) reflektierte Licht von den Detektorelementen (80) des Detektors (30) mehrmals, insbesondere höchstens fünfmal, erfasst wird, wodurch ein zweidimensionaler Schnitt (68) durch ein Raumelement (33) der Probe (1) in einer vorgegebenen Tiefe (T) der Probe (1) erhalten wird.

Fig. 1

Fig. 2 a)

Fig. 2 b)

Fig. 3 a)

Fig. 3 b)

EP 2 339 329 A2

Fig. 4

Fig. 5

Fig. 6 a)

Fig. 6 b)

Fig. 6 c)

Fig. 7

Fig. 8

Fig. 9 a)

Fig. 9 b)

Fig. 9 c)

Fig. 10 a)

Fig. 10 b)

EP 2 339 329 A2

Fig. 11

Fig. 12

Fig. 13

Fig. 14 a)

Fig. 14 b)

Fig. 15

Fig. 16

Fig. 17

Fig. 18

Fig. 19

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **AUS W. Y. OH et al.** *OPTICS EXPRESS,* 2006, vol. 14 (19), 8675-8684